(19) Europäisches Patentamt
European Patent Office
Office européen des brevets

(11) **EP 3 990 919 B1**

(12) **EUROPEAN PATENT SPECIFICATION**

(45) Date of publication and mention of the grant of the patent:
**13.09.2023 Bulletin 2023/37**

(21) Application number: **20733477.2**

(22) Date of filing: **23.06.2020**

(51) International Patent Classification (IPC):
**G01N 33/536** *(2006.01)*    **C12Q 1/6818** *(2018.01)*
**G16B 40/10** *(2019.01)*    **G16C 20/10** *(2019.01)*
**G16C 20/30** *(2019.01)*

(52) Cooperative Patent Classification (CPC):
(C-Sets available)
**G01N 33/536; C12Q 1/6818;** G16B 40/10;
G16C 20/10; G16C 20/30          (Cont.)

(86) International application number:
**PCT/EP2020/067493**

(87) International publication number:
**WO 2020/260277 (30.12.2020 Gazette 2020/53)**

(54) **METHOD AND KIT FOR MEASURING OF ANALYTES IN BI-COMPONENT SYSTEMS AND USES THEREOF**

VERFAHREN UND KIT ZUR MESSUNG VON ANALYTEN IN ZWEIKOMPONENTENSYSTEMEN UND DEREN VERWENDUNG

PROCÉDÉ ET KIT DE MESURE D'ANALYTES DANS DES SYSTÈMES À DEUX COMPOSANTS ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2019 EP 19182157**

(43) Date of publication of application:
**04.05.2022 Bulletin 2022/18**

(60) Divisional application:
**23189658.0**

(73) Proprietor: **Actome GmbH**
**79110 Freiburg (DE)**

(72) Inventors:
• **JENEY, Csaba**
**79379 Müllheim (DE)**
• **KOLTAY, Peter**
**79117 Freiburg (DE)**

(74) Representative: **Hertin und Partner Rechts- und Patentanwälte PartG mbB Kurfürstendamm 54/55 10707 Berlin (DE)**

(56) References cited:
**EP-A1- 1 460 414**    **EP-A1- 2 837 937**
**EP-A2- 0 576 879**    **WO-A1-2016/209869**
**WO-A1-2017/046284**    **CN-B- 106 226 516**
**JP-A- H06 109 740**

• **Y.T. Wu: "Förster resonance energy transfer immunoassays using engineered proteins for breast cancer biomarker detection", Ph.D. Thesis. Université Paris-Saclay , 31 October 2018 (2018-10-31), XP002796630, Retrieved from the Internet: URL:https://tel.archives-ouvertes.fr/tel-0 1909324 [retrieved on 2019-12-20]**

- BINDER HANS ET AL: ""Hook"-calibration of GeneChip-microarrays: Theory and algorithm", ALGORITHMS FOR MOLECULAR BIOLOGY, BIOMED CENTRAL LTD, LO, vol. 3, no. 1, 29 August 2008 (2008-08-29) , page 12, XP021045339, ISSN: 1748-7188, DOI: 10.1186/1748-7188-3-12
- Matthew T Laurie ET AL: "Simultaneous digital quantification and fluorescence-based size characterization of massively parallel sequencing libraries", BioTechniques, 1 August 2013 (2013-08-01), pages 61-67, XP055245092, England DOI: 10.2144/000114063 Retrieved from the Internet: URL:http://www.biotechniques.com/multimedia/archive/00229/BTN_A_000114063_O_229541a.pdf [retrieved on 2016-01-27]
- THOMAS P. NIEDRINGHAUS ET AL: "Landscape of Next-Generation Sequencing Technologies", ANALYTICAL CHEMISTRY, vol. 83, no. 12, 25 May 2011 (2011-05-25), pages 4327-4341, XP055158762, ISSN: 0003-2700, DOI: 10.1021/ac2010857
- ELIZABETH G. REY ET AL: "Mitigating the Hook Effect in Lateral Flow Sandwich Immunoassays Using Real-Time Reaction Kinetics", ANALYTICAL CHEMISTRY, vol. 89, no. 9, 2 May 2017 (2017-05-02), pages 5095-5100, XP055566188, US ISSN: 0003-2700, DOI: 10.1021/acs.analchem.7b00638
- ADELAJDA ZORBA ET AL: "Delineating the role of cooperativity in the design of potent PROTACs for BTK", PROCEEDINGS OF THE NATIONAL ACADEMY OF SCIENCES, vol. 115, no. 31, 16 July 2018 (2018-07-16), pages E7285-E7292, XP055673555, US ISSN: 0027-8424, DOI: 10.1073/pnas.1803662115
- EUGENE F. DOUGLASS ET AL: "A Comprehensive Mathematical Model for Three-Body Binding Equilibria", JOURNAL OF THE AMERICAN CHEMICAL SOCIETY, vol. 135, no. 16, 16 April 2013 (2013-04-16), pages 6092-6099, XP055673569, ISSN: 0002-7863, DOI: 10.1021/ja311795d
- Christine J. Rossant ET AL: "Versatility of homogeneous time-resolved fluorescence resonance energy transfer assays for biologic drug discovery", Journal of Biomolecular Screening Society for Laboratory Automation and Screening, 1 January 2015 (2015-01-01), pages 508-518, XP055673575, Retrieved from the Internet: URL:https://journals.sagepub.com/doi/pdf/10.1177/1087057114557464 [retrieved on 2020-03-04]
- YUFENG XIONG ET AL: "Development of a novel immunoassay to detect interactions with the transactivation domain of p53: application to screening of new drugs", SCIENTIFIC REPORTS, vol. 7, no. 1, 23 August 2017 (2017-08-23) , XP055673584, DOI: 10.1038/s41598-017-09574-7
- Michelle R Arkin ET AL: "Inhibition of Protein-Protein Interactions: Non-Cellular Assay Formats", ASSAY GUIDANCE MANUAL, 18 March 2012 (2012-03-18), XP055209089, Retrieved from the Internet: URL:http://www.ncbi.nlm.nih.gov/books/NBK92000/pdf/Bookshelf_NBK92000.pdf [retrieved on 2015-08-21]
- RODBARD D ET AL: "Kinetics of two-site immunoradiometric ('sandwich') assays-I", IMMUNOCHEMISTRY, PERGAMON, GB, vol. 15, no. 2, 1 February 1978 (1978-02-01), pages 71-76, XP023798517, ISSN: 0019-2791, DOI: 10.1016/0161-5890(78)90045-7 [retrieved on 1978-02-01]
- RODBARD D ET AL: "Kinetics of two-site immunoradiometric ('sandwich') assays-II", IMMUNOCHEMISTRY, PERGAMON, GB, vol. 15, no. 2, 1 February 1978 (1978-02-01), pages 77-82, XP023798518, ISSN: 0019-2791, DOI: 10.1016/0161-5890(78)90046-9 [retrieved on 1978-02-01]

(52) Cooperative Patent Classification (CPC): (Cont.)

C-Sets
C12Q 1/6818, C12Q 2531/113, C12Q 2535/122, C12Q 2563/131, C12Q 2563/159, C12Q 2563/179

**Description**

The present invention relates to a method and kit of measuring parameters of an analyte using bi-component detection methods and uses thereof. In one aspect, the present invention relates to the determination of the dissociation constants of analyte-specific binding components of bi-component methods. The claimed method relates to determination of dissociation constants of components and better quantitation of bi-component detection methods, also in a parallel manner.

BACKGROUND OF THE INVENTION

Technical Field

The present invention relates to methods and kits of measuring parameters of an analyte using bi-component detection method and uses thereof. Applications of the methods and kits are also disclosed.

Discussion of Related Art

In particular, the methods and kits are well suited for determining the dissociation constants of analyte binding components .

The saturation effect or "hook effect" is a phenomenon common to detection systems involving saturation of reagent components used to capture specific binding partners or analytes, generally. However, the mathematical background is different in the case of mono-component or bi-component detection methods.

Bi-component detection systems are typically exploited in homogeneous assays where two components are applied to produce detection signals. Many of these assays apply a proximity concept and the analyte and the components need to be brought in proximity to produce a signal. Proximity assay technologies such as FRET (fluorescence resonance energy transfer), BRET (bioluminescence resonance energy transfer) (Pfleger, Seeber, & Eidne, 2006), cyan fluorescent protein (CFP) - yellow fluorescent protein (YFP) pair, PCA (protein complementation assays) (Morell, Ventura, & Avilés, 2009), Alphascreen (Taouji, Dahan, Bosse, & Chevet, 2009), and DNA labeled proximity methods (PLA - proximity ligation assay, PEA - proximity extension assay) (Söderberg et al., 2006), and a not proximity based assay called emulsion coupling are representative examples of bimolecular (bi-component) detection systems and methods.

In mono-component detection systems, only one assay component, called the tracer, is required to produce a signal. Filtration binding assays involving radioactive or fluorescent tracers are good examples of mono-component detection systems. Standard curves obtained using mono-component detection systems show a typical sigmoid shape ending by a plateau (also commonly called 'hook') obtained at saturating tracer concentrations. These curves are also called saturations curves.

Standard curves produced with bimolecular detection systems are atypical. These curves are non-bijective and characterized by a signal decrease ('hook effect') following a plateau obtained after an initial concentration-dependent signal increase. The range of target molecules concentrations where the signal starts to drop is called the hook point. Below the hook point, the assay components become progressively saturated by the target molecule and a signal increase is measured. At the hook point, both components are saturated with the target molecule and a maximum signal is detected. Above the hook point, an excess of target molecules oversaturates the components, which inhibits their association and causes a progressive signal decrease.

In the prior art it is necessary to determine the hook point of a two-segment non-bijective standard curve before carrying out a measurement of an analyte concentration in order to ensure that all measurements are carried out in the range of a progressively saturated segment of the two-segment non-bijective standard curve. Over-saturation generally considered as a failure of measurement.

A failure of this requirement can result in false measurements since the determined values are not uniquely corresponding to an analyte concentration on the non-bijective standard curve.

In order to avoid such errors, typically the dynamic range of the measurement is limited to the dynamic range of the progressively saturated segment of the non-bijective standard curve.

In the prior art some approaches are known to address the Hook-Effect in particular in sandwich immunoassays.

Wu et al. 2018 discusses the occurrence of the Hook-Effect in a sandwich immunoassay due to an excess of capture and detection antibodies and suggests dilution of the sample to avoid misinterpretation of the results.

JP H06 109740 A relates to a turbidmetry immunoassay (TIA) for a quantitative analysis of target component in a sample using an antibody-antigen reaction. JP H06 109740 A discusses the occurrence of a prozone for an excess of antigen in known methods, which in such cases repeat the test in a sample dilution. As an improvement JP H06 109740 A suggests obtaining two or more calibration curves for samples having different concentrations and deciding which calibration curve is applicable based upon the deviation from a virtual curve. The disclosure of JP H06 109740 A is however conceptionally restricted to preventing the prozone effect to avoid false negative measurements, without allowing for a broader dynamic range of the assay from defined mathematical relationships.

EP 0 576 879 A2 relates to methods for determining the concentration of a component in a medical sample and discusses the Hook effect in immunological detection schemes at antigen excess as well a resulting non-monotonic, non-bijective calibration curve, which is referred to as a "Heidelberger-Kurve". EP 0 576 879 A2 discloses that it is known in the prior art to overcome the ambiguity of the non-monotonic calibration curve using two different sample dilutions. As a further improvement EP 0 576 879 A2 suggests the use of a learning algorithm and multivariate statistics to correlate a measured input value with a correct regime of a calibration curve. However, EP 0 576 879 A2 relates to the analysis of signaling in a precipitation assay and thus cannot be straightforward extended to bi-component assays, especially as precipitation is a variable/multi-component reaction with undefined stoichiometry. Moreover, using the learning algorithm the results may vary for different experimental conditions, such that different calibrations curves may, become necessary for each experimental condition.

CN 106 226 516 B relates to detection methods involving hyperbolic calibration curves and also discusses strategies to cope with the Hook effect. CN 106 226 516 B suggests performing separate reaction of standards of different concentration of analytes with equal amounts of reactant in order to assemble a calibration curve at the point of inflection from a pre-calibration and post-calibration curve.

Binder et al 2008 discloses calibration algorithms for a GeneChip microarray that may take into account a Hook-Effect and comprise co-processing the log difference and sum of perfect match and mismatch probe intensities.

Given the teaching of the prior art there is thus room for improvement for providing methods and kits suitable for determining the concentration of an analyte using bi-component detection assays, which may reliable, effectively and with few additional effort broaden the dynamic range of such bi-component detection assays and provide a defined mathematical relationship that eliminates the need for using approximate methods.

In another aspect, the methods and kits are suitable for determining the dissociation constants of analyte binding components using bi-component detection methods and relate to the measurement of dissociation constants in parallel manner using bi-component detection methods and uses thereof.

In chemistry, biochemistry, medical technologies and pharmacology, a dissociation constant (Kd) is a specific type of equilibrium constant that measures the propensity of a larger complex to dissociate reversibly into smaller components (Friguet, Chaffotte, Djavadi-Ohaniance, & Goldberg, 1985). The dissociation constant is the inverse of the association constant. In the special case of bi-component measurement methods, the dissociation constants are the dissociation constants between the two components and the analyte. As the components can be antibodies, the determination of the dissociation constants has a broad interest.

In the prior art some approaches are known for determining dissociation constants.

Rossant ET AL 2015 and Xiong Y. et al. (2017) disclose approaches for determining equilibrium parameters (Kd) of affinity complexes using FRET-based assays, but relate to binary complexes.

Rey et al. 2017 explore the role of cooperativity in the design of potent proteolysis targeting chimeras (PROTACs) for degradation of Bruton's tyrosine kinase (BTK). Rey et al. proposes that maximizing PROTAC activity on BTK is associated with maximizing ternary complex of BTK:PROTAC:CRBN and discloses a TR-FRET analysis of the said ternary complexes with members of a PROTAC library serving as an analyte and BTK and CRBN as ligands, which are each labeled with a FRET (donor/acceptor) fluorescent molecule. Rey et al. further discloses non-bijective curves for ternary complexes formation (and resulting FRET signal) over concentration of an analyte as well as numerical fitting for deriving dissociation constants ($K_D$ values) for the binding of PROTAC with BTK or CRBN.

EP 2 837 937 A1 discloses a mathematical model for the binding equilibria in ternary affinity complexes. EP 2 837 937 A1 discusses features of ternary complex formation, including the "bell-shaped" binding curve or "Hook effect". Equations for the relationships between equilibrium dissociations constants and concentrations of analyte, binding components and/or ternary complex concentrations are also disclosed.

While Rey et al. 2017 and EP 2 837 937 A1 address an estimation of equilibrium parameters in ternary complex formation and mention the occurrence of a Hook effect, they do not provide a solution to properly take into account these effects.

EP 1 460 414 A1 relates to homogenous time resolved FRET assay for the identification of potential lead antibodies in drug discovery processes. EP 1 460 414A1 discloses examples for using HFRET assay to analyze protein-protein interactions. As a disadvantage of direct binding assays EP 1 460 414 A1 discloses that screening of undiluted samples may increase the number of false negatives as a result of the "Hook" effect. Screening at multiple sample dilutions is proposed to limit the effect, but may affect time and costs.

Zorba et al. discloses principles of the amplified luminescent proximity homogenous assay (AlphaLISA) which was modified from a binding competition assay to detect the interactions between the transcription-activation domain p53 and its ligands. Zorba et al. discusses the occurrence of the Hook effect at exceeding concentrations and proposes suitable titration for avoidance.

Douglass et al. 2013 discuss different assays for monitoring protein-protein interaction and identifying small molecules as inhibitors including AlphaScreen or AlphaLISA. Douglass et al. also discusses the hook effect and suggest titrations to choose a protein concentration avoiding the hook point for the assay.

Neither EP 1 460 414 A1, Zorba nor Douglass et al. 2013 address however the estimation of dissociation constants in these assays.

In light of the teachings of the prior art it would be desirable to provide improved methods and kits suitable for measuring dissociation constants using bi-component detection methods, especially in in parallel manner or high throughput format.

Furthermore, methods for determining the dissociation constants of bi-component detection methods in the prior art are elaborate and expensive, however especially in parallel manner they are seriously limited. Most frequently used methods like displacement ELISA, fluorescence polarization method, and surface plasma resonance need the parallelization of standalone measurements. A need for a simplified and reaction-level parallel yet robust method therefore exists.

## DISCLOSURE OF THE INVENTION

In light of the prior art the technical problem underlying the present invention is to provide alternative and/or improved means for determining the dissociation constants of analyte-specific binding components in a bi-component detection method.

An object of the present invention is to provide for a method and kit of measuring dissociation constants of analyte-bi-component complexes using compartmentalized bi-component detection methods of measurement and uses thereof.

In particular, a purpose of the invention is to provide for a method of measuring dissociation constants of analyte-bi-component complexes and parallel detection in compartmentalized bi-component detection methods and uses thereof.

This object is solved by the features of the independent claims. Preferred embodiments of the present invention are provided by the dependent claims.

In one aspect the invention relates to a method for determining the dissociation constants of analyte-specific binding components in a bi-component detection method comprising:

a. providing two non-immobilized analyte-specific binding components at known concentrations for conducting a bi-component detection method in solution comprising bringing said two analyte-specific binding components in contact with a solution containing said analyte to produce a signal that depends on the concentration of bi-component/analyte complexes formed in said solution,

b. providing a dissociation constant relationship for said bi-component detection method which is a mathematical functions for the relationship between the dissociation constants of said analyte-specific binding components in dependence of said signal reflecting the concentration of bi-component/analyte complexes and the concentration of analytes and/or analyte-specific binding components,

c. preparing one or more dilutions of said sample or said analyte-specific binding components,

d. applying the bi-component detection method to the sample and the one or more dilutions,

e. determining the dissociations constants of said two analyte-specific binding component using said signal detected of the sample and the one or more dilutions, as constraining inputs for a mathematical fit for said dissociation constant relationship at different analyte concentrations and/or analyte-specific binding components concentrations.

The method of the invention allows the determination of dissociation constants for bi-component detection systems by preparing one or more dilutions of the sample with known dilution factors and applies the bi-component detection method to the sample as well as the one or more dilutions. Applying the bi-component detection method for determining the dissociation constants of analyte-specific binding components preferably means that two analyte-specific binding components at known concentrations are brought into contact with the sample at known concentration in order to produce a signal that depends on the concentration of bi-component/analyte complexes formed in said solution. Either the concentration of the analyte-specific binding components or the sample is varied by dilution providing dilutions with known dilution factors. The signal detected of in sample and in the one or more dilutions as well as the concentration of analytes and/or analyte-specific binding components can be used as a constraining input for the mathematical fit.

The two analyte-specific binding components as well as the analyte are non-immobilized and in solution, such that the bi-component/analyte complexes likewise form in solution. In some embodiments the method comprises a step of providing a sample with a known concentration of analyte. In some embodiments the method is an *in-vitro* method, wherein preferably a step of providing a sample does not comprise a surgical or invasive treatment of a living human or animal body. As detailed above the signal can take a variety of forms and depends on the bi-component detection system used. For instance, in case of proximity assays such as a FRET assay, the signal may refer to a fluorescence signal from a FRET-donor-acceptor pair indicating the formation bi-component/analyte complexes. In case of a compartmentalized bi-component method such as emulsion coupling, the signal may also refer to signals resulting from a ddPCR and/or NGS-sequencing that indicate the presence of a bi-component/analyte complex based upon a deviation from the expected Poisson distribution in the compartmentalized droplets.

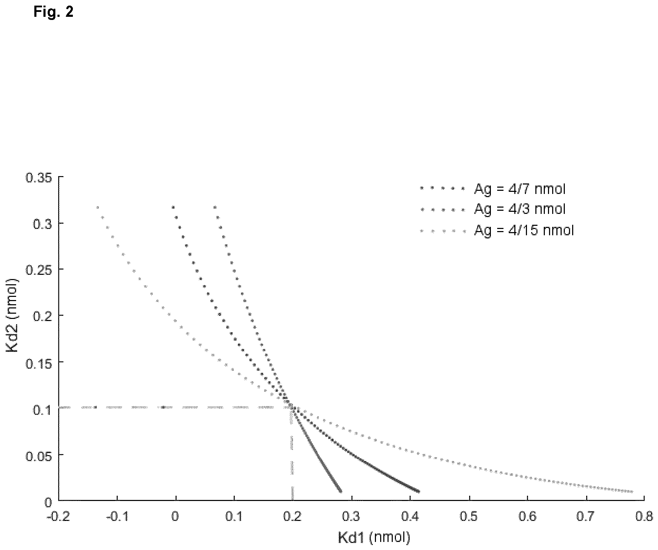

As shown in the examples, (see e.g. Example 2, 5 and Fig. 2) using the signal detected in the sample and in one or more dilutions as a constraining input for a mathematical fit allows for a robust determination of dissociations constants of two analyte-specific binding components with the analyte.

The dissociation constant relationship may be provided by analytically solving chemical balance, and mass conservation equations (see Example 2; Eq. 3). In generally terms, it can be described as f(Kd1,c12,a0)=Kd2, wherein "Kd1" and "Kd2" are dissociation constants, "c12" is the measured concentration of ternary complex, as determined by the signals reflecting the formation of bi-component/analyte complexes and "a0" is the known concentration of the analyte. As a further input f(Kd1 ,c12,a0)=Kd2 may further comprises the concentration of analyte, end both bi-components, which are also known. These terms are valid in the case of known and not varied concentration of analyte-specific binding components.

Another advantageous aspect of determination of dissociation constant relationship may be provided if the concentration of analyte-specific binding components are varied, it can be described as f(Kd1,c12, b10, b20)=Kd2, wherein "Kd1" and "Kd2" are dissociation constants, "c12" is the measured concentration of ternary complex, as determined by the signals reflecting the formation of bi-component/analyte complexes and "b10" and "b20" are the known concentration of the analyte-specific binding components. As a further input f(Kd1,c12,b10,b20)=Kd2 may further comprises the concentration of analyte, which is also known. These terms are valid in the case of known and not varied concentration of analyte.

In order to determine the dissociation constants ("Kd1" and "Kd2") of the binding components Eq 3. may be applied. Having more than one solution containing a known amount of analyte, bi-components and a determined amount of ternary antibody/bi-component complex, the two Kd can be calculated according to the Example 2.

As different dilutions of the sample containing the analyte corresponds to different concentration of "c12", the "f" function has different forms (see Fig. 2). However since the Kds are material constants, all forms of the "f" function must be satisfied by a certain pair of values of Kds.

In practice, graphing the dissociation constant relationships according to Kd1 and Kd2 , the curves of the different known concentrations intersect close to one point which are the applicable pair of Kds to be determined (see Example 2). In other words, the mathematical solutions are preferably to find an intersection between of curves, which reflect the dissociation constants (Kd1-Kd2) relationships varied by the different input concentrations as provided by applying the bi-component detection method to the sample as well as one or more dilutions.

In one embodiment determining more than one relationship of the dissociation constants using varied experimental conditions, e.g. by providing different dilutions of the analyte, can help to determine the value of the dissociation constants, which satisfy these relationships.

In a preferred embodiment, one can determine the value of the dissociation constants using statistical models and/or other mathematical tools, since measurement errors are confounded in different measurements.

In another aspect, the methods are suitable for determining the dissociation constants of analyte binding components using a bi-component detection method in parallel manner using bi-component detection methods and uses thereof.

In one embodiment, the methods for determining the dissociation constants of analyte binding components is based on absolute quantifiable compartmentalized bi-component detection methods.

Atypical compartmentalized method is the digital PCR (ddPCR) (Quan, Sauzade, & Brouzes, 2018) method utilizing a water-oil emulsion droplet system. Droplets are formed in a water-oil emulsion to form partitions (compartments) that separate the template DNA molecules. The droplets serve essentially the same function as individual test tubes or wells in a plate in which the PCR reaction takes place. The massive sample partitioning is a key aspect of the ddPCR technique.

The ddPCR technology is digital, in the regard that, the droplets support PCR amplification of the template molecules they contain and generate signals based on individual DNA template molecules. Following PCR, each droplet is analyzed or read to determine the fraction of PCR-positive droplets in the original sample. These data are then analyzed using Poisson statistics to determine the absolute DNA template concentration in the original sample.

In another embodiment the method may be used to apply the absolute quantitation principle (Quan et al., 2018) to bi-component measurement methods. The signal of the analyte is based on detection of the individual molecules of the compartmentalized analyte-bi-component complexes and Poisson statistics, wherein the analyte-bi-component complexes are determined by the chemical balances based on the concentration of the molecules and their analytical properties, namely dissociation constants.

In this embodiment, the creation of tens, thousands or millions (or even higher number) of droplets and their application for bi-competent detection method means that a single sample measurement having the number of droplets as the primary dynamic range of the bi-component method, in a absolute quantitative, highly linear manner, rather than using a single, inherently non-linear signal as in the case of non-compartmentalized measurements obtained. Thereby bringing the absolute quantitativity and statistical features inherent to compartmentalized methods into the detection of concentration of analytes using bi-competent methods.

In some embodiments, these methods are preferred for multi-analyte measurements as they usually have vast concentration differences. In such embodiments one can measure DNA copy number (low-abundance) and RNA/protein copy number (high abundance) in the same sample using suitable compartmentalized bi-component method such as emulsion coupling (see examples below and EP 3224360).

In another embodiment of the methods described herein, it is preferred to use parallel reading technologies. Next-generation DNA sequencing is preferred in some embodiments as a bi-component readout technology. Particularly those bi-component methods are suitable for DNA sequencing based readout, which generate unique DNA signals as a part of the detection principles. These methods include, but are not limited to proximity ligation, extension assay and emulsion coupling.

In another embodiment DNA sequencing readout based bi-component measurement methods are preferred, if the bi-component methods is a compartmentalized bi-component method. Under compartmentalized conditions, the generation of unique DNA signals is unbiased, compared to the uncompartmentalized measurements.

In another aspect of compartmentalized generation of DNA signals means unbiased DNA signals that enabled by the usage of unique molecular identifiers (UMI) (Parekh, Ziegenhain, Vieth, Enard, & Hellmann, 2017) signal readout.

Various methods have been developed to improve accuracy of quantification of different polynucleotides in a sample using next-generation sequencing technologies, including such methods as competitive polymerase chain reaction (PCR), described in U.S. Patent Number 5,213,961 and deep barcode sequencing using unique molecular identifiers (UMI), as described by (Smith et al., 2009).

Unique molecular identifiers, or molecular barcodes, provide an advantage in quantifying unique DNA signals of the bi-component measurement methods in a sample. However, if UMIs are involved in more than the first round of detection, the same UMI may be introduced into different targets, resulting in counting errors. Also, the original UMI method is based on an ideal, but unrealistic, situation- that is, where both PCR and sequencing technologies are both perfect and no errors are introduced. The UMI strategy operates on the assumption that both PCR and sequencing steps report the underlying targets and UMI fragments free-of-error. These conditions are difficult to provide under uncompartmentalized conditions, but seamlessly provided under compartmentalized conditions. UMI labeled components are single or few molecules per compartment in a compartmentalized bi-component measurement method, which means effective incorporation of the UMI barcodes into the unique DNA signals.

In another aspect, unique molecular identifiers (UMIs) representing unique DNA signals can be incorporated into unique DNA signals and further amplified in a compartmentalized bi-component method. However, without the usage of the unique molecular identifiers, the quantitation of the unique DNA signal is biased. The amplification can be specific to certain unique DNA signals; to balance their ratio against other not further amplified unique DNA signals.

In another aspect, unique molecular identifiers incorporated unique DNA signals can be used to detect analytes with two or more identical binding sites or analytes with more than two non-identical binding sites. These arrangements are particularly preferred to detect interacting analytes, like proteins or other molecules.

In another aspect, the methods and kits of bi-component measurement methods using parallel reading technologies, can measure both the concentration of analyte and dissociation constants of the components in a parallel manner. Today next-generation sequencing technologies can provide billions of independent reads, which are directly proportional with the achievable parallelism of the readout.

In one further embodiment, the analyte is selected from a group consisting of proteins, peptides, nucleic acid segments, carbohydrates, lipids, antibodies (monoclonal or polyclonal), antigens, oligonucleotides, specific receptor proteins, ligands, molecules, cells, microorganisms as well as fragments products or combinations of thereof.

In one embodiment the two analyte-specific binding components are selected from a group consisting of nucleic acids, preferably RNA and/or DNA oligonucleotides, antibodies, peptides, proteins, aptamers, molecularly-imprinted polymers, cells or combinations of thereof.

In one embodiment, the sample comprise two or more different types of analytes.

In one embodiment, two or more pairs of different kind of analyte-specific binding components are used.

A particular advantage of the methods described herein is that it is even possible to have more than one kind of analytes (e.g. DNA; RNA; protein, interacting proteins and their chemical modifications) and similarly more than one kind of binding component in the same assay.

Using parallel approaches as described herein, the analyte concentration for different kind of analytes can be determined in parallel as well as dissociation constants for different kind of analyte-specific binding components.

In one embodiment the bi-component method comprises employing a proximity-based assay to produce the bi-component/analyte complexes concentration depended signal, wherein the proximity-based assay uses two analyte-specific binding components that produce a detectable signal in dependence of their proximity.

In one embodiment the bi-component method comprises employing a resonance energy transfer assay, preferably a Förster resonance energy transfer (FRET) assay or a bioluminescence resonance energy transfer (BRET) assay, a protein complementation assay (PCA), Alphascreen or a DNA labeled proximity assay, preferably a proximity ligation assay (PLA) or a proximity extension assay (PEA).

In one embodiment, the bi-component detection method comprises employing a compartmentalized assay to produce a bi-component/analyte complexes concentration depended signal, wherein the signal reflects the presence of said two analyte-specific binding components in a single compartment.

In one embodiment, the compartmentalized assay employs an emulsion droplet method, wherein each droplet in the emulsion represents a separate compartment.

In one embodiment, the compartmentalized assay is emulsion coupling. As described in detail below emulsion coupling refers to is a digital assay concept based on the detection of double-labelled (bi-component), individual ternary molecular complexes in emulsion, which may be identified, for example, by digital droplet PCR (ddPCR) using fluorescently tagged PCR products or next generation sequencing (NGS).

Advantageously, such an approach allows for an absolute quantification of analyte concentration as well as a parallel determination of the concentration of multiple analytes in a robust and efficient manner.

In one embodiment, the bi-component detection method comprises employing an absolute molecular count based analytical method. Such an absolute molecular count based analytical method preferably refers to applying digital detection methods, such as a digital PCR or next-generation sequencing.

In one embodiment, the bi-component detection method comprises employing a droplet digital PCR assay.

In one embodiment, the bi-component detection method comprises using analyte-specific binding components associated with unique amplifiable nucleic acid labels and employs a compartmentalized assay, wherein a nucleic acid amplification is performed for each compartment using fluorescently tagged amplification products.

In preferred embodiments, the nucleic acid amplification is a PCR and the fluorescently tagged amplification products are fluorescently tagged PCR products.

The analyte-specific binding components (such as an antibody pair) may preferably be labelled by unique PCR amplifiable DNA labels, that is the two analyte-specific binding components, e.g. two antibodies, may preferably be labeled with a single stranded DNA that uniquely identifies the binding component (e.g. antibody). The labelled binding components (e.g. antibodies) are added to the sample or the one or more dilutions in order to allow for bi-component/antibody complex formation.

Subsequently the reaction is preferably highly diluted, e.g. by a dilution factor of more than 20 000, preferably more than 50 000, 100 000 to achieve single-complex separation upon a compartmentalization, e.g. by emulsification into droplets.

For the nucleic acid amplification, fluorescently tagged amplification products are used that recognize the binding component (e.g. antibody) specific unique amplifiable nucleic acid labels. For instance, fluorescently tagged PCR products, e.g. using FAM- or VIC-labelled real-time PCR probes may be used that are complementary to the single stranded DNA that uniquely identify the binding component.

The nucleic acid amplification is performed in each of the compartments, e.g. emulsion droplets and a signal readout may be performed by detecting the fluorescence single of the compartments, e.g. the 'color' of the droplets.

Preferably, ddPCR is employed and according to a standard evaluation of ddPCR, the clusters of droplets may determine according to the fluorescent signals of the droplets. Herein, the number of labelled binding components (e.g. antibodies) is determined in each reaction (counting all label-positive droplets for a given label, and using the same definition of cluster of droplets for all reactions). Additionally the number of the double-colored (having two different binding component labels) is also determined.

Without the formation of ternary bi-component/analyte complexes, the partitioning of the labelled antibodies follows Poisson distribution, and results in a calculable number of double-colored droplets (having two binding components in one compartment based upon pure chance). In the case of ternary complexes present in the reaction, the number of the detected double-colored droplets (having additional ternary complexes) is larger than would be expected by Poisson distribution alone. Based upon such an analysis the number of ternary complexes can be thus calculated. Advantageously, the embodiment allows for an absolute quantitation of the ternary analyte complexes formed.

In one embodiment of the method described herein, multiple analytes are determined in parallel.

In one embodiment the bi-component detection method comprises using multiple analyte-specific binding components comprising nucleic acid barcodes and employs a compartmentalized assay, wherein a nucleic acid amplification is performed for each compartment producing linked nucleic acid barcodes, the compartments are reunited in a common pool and a parallel nucleic acid sequencing technique is used to produce the bi-component/analyte complexes concentration depended signal.

A preferred parallel nucleic acid sequencing technique used herein is a next generation sequencing technique.

In preferred embodiments, the binding components comprising nucleic acid barcodes refer to antibodies labelled with unique PCR amplifiable DNA labels, comprising a unique label for the type of antibody termed and also a label for the individual molecule (unique molecular identifier - UMI) (see also Parekh et al., 2017). The thus labelled antibodies are added to the sample in order to allow for bi-component/antibody complex formation.

Performing the nucleic acid amplification for each compartment to produce linked nucleic acid barcodes may be achieved by highly diluting the sample before the nucleic acid amplification, e.g. with a dilution factor of more than 20 000, preferably more than 50 000, more preferably more than 100 000. In preferred embodiments, the nucleic acid amplification is PCR. PCR reagents may be added to achieve single-complex separation and nucleic acid amplification per water-in-oil emulsion droplet. To this end droplet digital PCR standard protocols may be particularly suited.

Afterwards the compartments, e.g. emulsion droplets, may be recombined in a common pool and a parallel nucleic acid sequencing technique can be used to assess antibody specific dimerized UMI labels. Herein, the number of labelled antibodies can be determined in each reaction by counting all unique UMI labels for a given antibody (counting restricted to a given antibody - e.g. in a given label context). A possible multiple labeling of the same antibody can be eliminated using their preferentially dimerized sequences, since multiple labels per antibody exhibit a double UMI label dimers with a given antibody specific label context, as they are co-localizing in the same droplet.

The ternary bi-component/antibody complexes can be counted on the basis of their dimerized double UMI labeled PCR products two different antibody specific labels, called heterodimers) with a correction according to the antibody multiple labels.

Without the formation of ternary complexes, the partitioning of the labelled antibodies follows Poisson distribution, and results in a calculable number of ternary complexes (based on the detection of heterodimers) in droplets. In the case of ternary complexes present in the reaction, the number of the detected heterodimers is larger than would be expected by pure Poisson distribution. Based on this measurement the number of complexes can be calculated.

In a compartmentalized bi-component method, it is advantageous to incorporate UMIs into different targets as an encoding step for co-localization of unique DNA signals.

In a further preferred embodiment, the invention relates to a kit, preferably for carrying out the method for determining the dissociation constants in a bi-component detection method as described herein, comprising:

a. reaction buffer for two analyte-specific binding components at known concentrations for conducting a bi-component detection method comprising bringing said two analyte-specific binding components in contact with an analyte to produce a signal that depends on the concentration of bi-component/analyte complexes formed in said solution,

b. reference data on a dissociation constant relationship for said bi-component detection method which is a mathematical functions for the relationship between the dissociation constants of analyte-specific binding components (kd1 and Kd2, respectively) with the analyte in dependence of said signal reflecting the concentration of bi-component/analyte complexes and the concentration of analytes and/or analyte specific binding components,

c. optionally instructions for preparing one or more dilutions of said sample and applying the bi-component detection method to the sample and the one or more dilutions using defined dilutions factors,

d. a computer program when executed on a computer configured for performing the computational steps of determining the dissociations constants kd1 and kd2 of said two analyte-specific binding component using said signal detected in the sample and in the one or more dilutions as a constraining input for a mathematical fit for said dissociation constant relationship at given analyte and/ or analyte specific binding components concentrations.

Technical features that have been disclosed for the methods described herein also apply for the kits for use in such methods. A person skilled in the art will, therefore, recognize that preferred features of the methods described herein can be advantageously used as well in the context of the kits.

The method of the present invention, in some embodiments, may also relate to a computer programme product, such as a software product.

The software may be configured for execution on common computing devices and is configured for carrying out one or more of steps of the method described herein.

In one embodiment the computer program may be configured for carrying out step e) of claim 1 that is performing the computational steps of determining the dissociations constants kd1 and kd2 of said two analyte-specific binding component using said signal detected in the sample and in the one or more dilutions as a constraining input for a mathematical fit for said dissociation constant relationship at given binding components and analyte concentrations or further preferred embodiments of the computational steps as disclosed herein.

In one embodiment a dissociation constant relationship for the bi-component detection method may be provided in form of reference data.

Herein, reference data for a dissociation constant relationship for a bi-component detection method shall preferably relate to any data that allows for providing a mathematical function for the relationship between the dissociation constants of the analyte-specific binding components (kd1 and kd2, respectively) with the analyte in dependence of the signal reflecting the concentration of bi-component/analyte complexes and the concentration of analytes and/or analyte specific binding components-

Typically, the reference data may be stored on a computer-usable or computer-readable medium on the control unit. Any format may be suitable which is used in the industry. The reference data may be stored in a separate file and/or integrated in the computer code or software (e.g. in the source code) for performing the computational steps for the dissociations constants kd1 and kd2, as described above.

The computer program product or kit comprising the computer program of the present invention therefore also encompasses and directly relates to the features as described for the method provided herein. Further details on preferred computer-based approaches are provided in the examples and relevant references as described herein.

## DETAILED DESCRIPTION OF THE INVENTION

Before the present invention is described with regards to the examples, it is to be understood that the terminology used herein is for the purpose of describing particular embodiments only and is not intended to limit the scope of the present invention.

The term "sample" or "sample solution" as used herein refers to a solution comprising analytes, preferably of an unknown concentration. Examples of samples include biological fluids such as serum, plasma, urine, tear, cells, cell mixtures, cell culture supernatants, or cell lysates containing one or more biological target molecules. Furthermore, samples can also comprise any conditioning reagents (e.g., permeabilising reagents) needed to render analytes soluble and accessible to detection and quantification. Such conditioning reagents may be added to the sample at any time before or after conduction the methods described herein.

The term "analyte" refers to a substance to be detected, quantified or otherwise assayed by the method of the present invention. Typical analytes may include, but are not limited to proteins, peptides, nucleic acid segments, carbohydrates, lipids, antibodies (monoclonal or polyclonal), antigens, oligonucleotides, specific receptor proteins, ligands, molecules, cells, microorganisms, fragments, products and combinations thereof, or any substance for which attachment sites, binding members or receptors (such as antibodies) can be developed. Analytes may refer also to complexes from said entities. For instance, an analyte may refer to an aggregate or complex formed of multiple entities or molecules, e.g. a protein-protein complex, wherein it is the interaction of the entities/molecules is of interest.

By "protein", a sequence of amino acids is meant for which the chain length is sufficient to produce the higher levels of tertiary and/or quaternary structure. "Peptides" preferably refer to smaller molecular weight proteins.

The term "nucleic acid" refers to any nucleic acid molecule, including, without limitation, DNA, RNA and hybrids or modified variants and polymers ("polynucleotides") thereof in either single- or doublestranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid molecule/polynucleotide also implicitly encompasses conservatively modified variants thereof (e.g. degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19: 5081 (1991); Ohtsuka et al., J. Biol. Chem. 260: 2605-2608 (1985); Rossolini et al., Mol. Cell. Probes 8: 91-98 (1994)). Nucleotides are indicated by their bases by the following standard abbreviations: adenine (A), cytosine (C), thymine (T), and guanine (G).

As used herein, the term "nucleic acid amplification" refers to a process by which a limited quantity of nucleic acid undergoes a biochemical reaction in which a larger quantity of nucleic acid is generated. Nucleic acid amplification thus relates to the generation of additional copies of a nucleic acid sequence and is generally carried out using polymerase chain reaction (PCR) or ligase chain reaction (LCR) or other technologies well known in the art (see, for example, Dieffenbach, C. W. and G. S. Dveksler (1995) PCR Primer, a Laboratory Manual, Cold Spring Harbor Press, Plainview, N.Y.).

As used, herein the term "bi-component detection method" or "bi-molecular detection method" refers to a method using two analyte-specific binding components and determining a signal reflecting the formation of bi-component/analyte complexes, when said two analyte-specific binding components are brought into contact with a solution containing the analyte. The term "bi-component detection system" or "bi-molecular detection system", preferably refer to components or reagents necessary to carry out a bi-component detection method. This encompasses preferably two analyte-specific binding components, preferably provided in a single or separate solution at a known concentration as well as optional a sample solution containing the analyte to be analyzed.

Preferably the two analyte-specific binding components as well as the analyte are provided non-immobilized, i.e. in solution, such that the of bi-component/analyte complexes likewise form in solution. The term bi-component detection method as used herein, thus preferably address a liquid phase formation of ternary complexes and is distinct from common sandwich immunoassays involving as a solid phase immobilized (primary) capture binding agents as well (secondary) detection binding agents.

The term "non-immobilized" thus preferably refers to components, such as the analyte-specific binding components, that may freely diffuse within a (liquid) solution, such that the binding kinetics to the analyte, equally freely diffusing in said liquid solution, are governed by equations for law of conservation of mass and law-of-mass action in solution as described herein.

The term "analyte-specific binding components" or "analyte specific-binding partners" preferably refers to one member of a binding pair, wherein the second member is the analyte and wherein the term "binding pair" includes any of the class of immune-type binding pairs, such as antigen/antibody or hapten/anti-hapten systems; and also any of the class of nonimmune-type binding pairs, such as biotin/avidin; biotin/streptavidin; folic acid/folate binding protein; complementary nucleic acid segments such as complementary DNA strands or complementary RNA strands; protein A or G/immunoglobulins; and binding pairs which form covalent bonds, such as sulfhydryl reactive groups including maleimides and haloacetyl derivatives, and amine reactive groups such as isotriocyanates, succinimidyl esters and sulfonyl halides.

The binding components of the bi-component detection method preferably may refer to any component having a binding potential to the analyte. In preferred embodiments the analyte-specific binding components may be nucleic acids, preferably RNA and/or DNA oligonucleotides, antibodies, peptides, proteins, aptamers, molecularly-imprinted polymers, cells or combinations of thereof.

The term "antibody" as used herein covers monoclonal antibodies (including full-length monoclonal antibodies), polyclonal antibodies, multispecific antibodies (e.g., bispecific antibodies), and antibody fragments so long as they exhibit the desired biological activity.

"Antibody fragments" comprise a portion of a full-length antibody, generally the antigen binding or variable region thereof. Examples of antibody fragments include Fab, Fab', $F(ab')_2$, and Fv fragments; diabodies; linear antibodies; single-chain antibody molecules; and multispecific antibodies formed from antibody fragments.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see, e.g., U.S. Pat. No. 4,816,567). The "monoclonal antibodies" may also be isolated from phage antibody libraries using the techniques described in Clackson et al, Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. NatL. Acad Sci. USA 81:6851-6855 (1984)).

Complementarity determining regions (CDRs) are part of the variable chains in immunoglobulins (antibodies), generated by B-cells, where these molecules bind to their specific antigen. As the most variable parts of the molecules, CDRs are crucial to the diversity of antigen specificities generated by immunoglobulins. There are three CDRs (CDR1, CDR2 and CDR3), arranged nonconsecutively, on the amino acid sequence of a variable domain of an immunoglobulin. Since the immunoglobulins are typically composed of two variable domains (on two different polypeptide chains, heavy and light chain), there are six CDRs for each antigen receptor that can collectively come into contact with the antigen.

In addition, fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment (Ward, E.S. et ai, Nature 341:544-546 (1989)) which consists of a VH domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et ai , Science 242:423-426 (1988); Huston et al , PNAS USA 85:5879-5883 (1988)); (viii) bispecific single chain Fv dimers (PCT/US92/09965) and (ix) "diabodies", multivalent or multispecific fragments constructed by gene fusion (WO94/1 3804; P. Hollinger et ai, Proc. Natl. Acad. Sci. USA 90: 6444-6448 ( 1993)).

An "antigen binding domain" is the part of an antibody which comprises the area that specifically binds to and is complementary to part or all of an antigen. Where an antigen is large, an antibody may only bind to a particular part of the antigen, which part is termed an epitope. An antigen-binding domain may be provided by one or more antibody variable domains. An antigen-binding domain may comprise an antibody light chain variable region (VL) and an antibody heavy chain variable region (VH).

In some embodiments, the binding components may be based on engineered protein scaffolds. Protein scaffolds are derived from stable, soluble, natural protein structures that have been modified to provide a binding site for a target molecule, i.e. an analyte, of interest. Examples of engineered protein scaffolds include, but are not limited to, affibodies, which are based on the Z-domain of staphylococcal protein A that provides a binding interface on two of its a-helices (Nygren, P. A. (2008). FEBS J 275(1 1): 2668- 76); anticalins, derived from lipocalins, that incorporate binding sites for small ligands at the open end of a beta-barrel fold (Skerra, A. (2008) FEBS J 275(1 1) : 2677-83), nanobodies, and DARPins. Engineered protein scaffolds are typically targeted to bind the same antigenic proteins as antibodies. Short peptides may also be used to bind a target protein. Phylomers are natural structured peptides derived from bacterial genomes. Such peptides represent a diverse array of protein structural folds and can be used to inhibit/disrupt protein-protein interactions *in vivo* (Watt, P. M. (2006). Nat Biotechnol 24(2): 177-83)].

In the bi-component detection methods, preferably two analyte-specific binding components are used that both exhibit a binding potential for the analyte. Preferably, the two analyte-specific binding components do not compete with each other for the binding to the analyte, but are chosen and designed to allow for a simultaneous binding of the components to form bi-component/analyte complexes. As stated previously, the analyte may refer to an aggregate or complex formed of multiple entities, e.g. a protein-protein complex. One of the two analyte-specific binding components may bind to one entity of the analyte complex e.g. a first protein, and the other to a second entity of the analyte, e.g. a second protein. Thereby a bi-component/analyte complex is only form in case of interaction of both proteins, which in turn are labelled with the binding components.

In some embodiments, the binding components may be aptamers. Aptamers are synthetic oligonucleotides (DNA or RNA) that recognize target molecules with high affinity and specificity through a combination of shape complementarity and non-covalent chemical bonds (Blank & Blind, Current Opin. Chem. Biol., 2005, 9:336-342). These artificial ligands are obtained *in vitro* and can be developed to recognize a large variety of different molecule classes that range from mere ions (e.g. $Pb^{2+}$, Liu & Lu, 2003. J Am Chem Soc, 125, 6642-6643) to nucleotides, small molecules, proteins, viruses, and cells up to whole organisms (Menger et al., 2006. Handbook of Experimental Pharmacology, 359-373). High binding affinity aptamers have been selected through the well-known SELEX method (Ellington & Szostak, 1990. Nature, 346, 818-822) for the detection of low molecular weight molecules like theophyllin (Jenison et al., 1994. Science, 263, 1425-1429), L-arginine (Geiger et al., 1996. Nucl. Acids Res., 24, 1029-1036), moenomycin (Schuerer et al., 2001. Bioorg. Med. Chem., 92, 2557-2563), 17b-estradiol (Kim et al., 2007. Biosens. Bioelectron., 22, 2525-2531) but also for larger molecules like thrombin (thrombin-binding aptamer:5'-GGTTGGTGTGGTTGG-*3'*) (Baldrich et al., Anal Chem. 2004, 76, 23, 7053-63), cholera toxin or HIV-1 tat protein, among others (for review see Tombelli et al., 2007, Biomolec Eng., 24, 191-200).

Some of the above-mentioned aptamers have been used in ELISA-like assays on microplates or on the surface of biosensor transducers (QCM, SPR). An aptamer-modified AuNP colorimetric system has also been developed for the determination of the protein PDGF in a sandwich-based assay (Huang et al., 2005, 77, 5735-5741).

As used herein the term "analyte/bi-component complexes" or "bi-component/analyte complexes" refer to a ternary complex containing the analyte and both analyte-specific binding components. Such bi-component/analyte complexes are formed when applying a bi-component detection method to a solution containing an analyte. More specifically, the formation of bi-component/analyte complexes in a bi-component detection reflects directly upon the presence (and concentration) of an analyte in a given solution. In order to use the formation of bi-component/analyte complexes in order to determine the actual concentration of an analyte a signal reflecting the ternary complex formation is to be acquired.

As used herein the expression "signal that depends on the concentration of bi-component/analyte complexes" or "signal reflecting the formation of bi-component/analyte complexes" refers to any quantifiable information that allows for deduce the quantity of ternary bi-component/analyte complexes formed.

Naturally, the signal will depend on the applied bi-component detection method. For instance, if a Förster resonance energy transfer (FRET) assay is employed the signal may refer to the fluorescence signal that a FRET acceptor will emit when a FRET donor absorbs an excitation beam in close proximity. In the bi-component system, the FRET-acceptor or FRET-donor are preferably coupled the two analyte-specific binding components. Since a fluorescence signal from the FRET-donor-acceptor thus only when the respective binding components are in close proximity, the signal directly reflects upon the formation of ternary bi-component/analyte complexes.

Similarly, Alphascreen (Amplified Luminescent Proximity Homogeneous Assay) developed by PerkinElmer and commercially widely available is a further prototype bi-component detection method, where the signal generation relies on the proximity of "donor" and "acceptor" beads coated with a layer of hydrogel providing functional groups for bioconjugation. When a biological interaction between molecules brings the beads into proximity, a cascade of chemical reactions is initiated to produce a greatly amplified signal. Upon laser excitation, a photosensitizer in the "donor" bead converts ambient oxygen to a more excited singlet state. The singlet state oxygen molecules diffuse across to react with a chemiluminescer in the "acceptor" bead that further activates fluorophores contained within the same bead. The fluorophores subsequently emit light at 520-620 nm. Functional groups on the beads may represent the two analyte-specific binding components, e.g. in form of specific antibodies. Only when ternary complex containing the analyte and the two analyte-specific components are for formed the donor and acceptor beads are in close proximity resulting in a detectable fluorescent signal.

Due to the dependency of the FRET-assay or the Alphascreen assay on the proximity of the donor and acceptor and thus the analyte-specific binding components such assays are termed proximity based detection assays. Many different proximity based assays in the context of bi-component detections methods are known in the art and may be employed in the methods described herein.

As used herein the term "proximity based bi-component detection method" shall refer to any assay wherein the signal reflecting the formation of bi-component/analyte complexes is based upon the proximity of the two analyte-specific binding components. Statistically, the two analyte-specific binding components will likely be in close proximity in a solution to yield a detectable signal, if they form part of the same ternary complex.

Non-limiting examples of proximity based bi-component methods comprise methods employing a resonance energy transfer assay, preferably a Förster resonance energy transfer (FRET) assay or a bioluminescence resonance energy transfer (BRET) assay, a protein complementation assay (PCA), Alphascreen or a DNA labeled proximity assay, preferably a proximity ligation assay (PLA) or a proximity extension assay (PEA).

These proximity assay technologies are well known in the art. For further reference on FRET or BRET see e.g. (Pfleger, Seeber, & Eidne, 2006), for a protein complementation assays Morell, Ventura, &Avil6s, 2009, for Alphascreen Taouji, Dahan, Bosse, & Chevet, 2009 and for DNA labeled proximity methods such as PLA (proximity ligation assay) or PEA (proximity extension assay) Söderberg et al., 2006.

Proximity based assays are particularly preferred for "non-compartmentalized bi-component detection methods", which has used herein shall refer to bi-component detection methods, wherein no compartmentalization of the sample solution is conducted upon applying the bi-component detection method. Some non-compartmentalized assays may also be referred to as homogenous assays. In other words, the non-compartmentalized bi-component method preferably comprises the step of bringing the analyte and the bi-components in contact in a homogenous reaction solution and the detection step in order to acquire the signal reflecting the formation of ternary bi-component/analyte complexes can be performed upon the homogeneous sample solution without the separation of unbound components or analyte.

In particular preferred embodiments the methods as described herein uses compartmentalized bi-component detection methods. As used herein the term "compartmentalized bi-component detection methods" shall refer to bi-component detection methods that compartmentalize the sample after bringing the analyte specific binding components in contact with the analyte to quantify the formation of ternary analyte/binding-components.

In general, the compartmentalized bi-component detection methods are based upon compartmentalizing a solution to small compartments such that without the formation of ternary analyte/binding-component methods the presence of both binding components in the compartment is unlikely and follows a Poisson distribution.

For the compartmentalization, different assays can be envisioned. For instance, an emulsion droplet method may be used to form droplets in an emulsion (e.g. water-in-oil), wherein each droplet represents a separate compartment. Compartmentalization can involve locations or physical compartments, or diffusion limited environment.

The term of a "droplet" as used herein preferably refers to an isolated portion of a first fluid surrounded by a second fluid. The first fluid comprises preferably a hydrophilic fluid such as water, an aqueous media, or a buffer, and preferably comprises the sample solution or the one or more dilutions thereof to which the bi-component detections system or other reagents are added. The second fluid preferably a hydrophobic fluid, such as hydrocarbons, silicone oils, mineral oils, organic solvent. Emulsions techniques to compartmentalize sample solutions are well known in the art.

In particularly preferred embodiments the compartmentalized bi-component detection method is emulsion coupling, which refers to is a digital assay concept based on the detection of double-labelled (bi-component), individual ternary molecular complexes in emulsion, which may be identified, for example, by droplet digital PCR (ddPCR) or next generation sequencing (NGS).

Droplet Digital PCR (ddPCR) preferably refers to a method for performing digital PCR that is based on water-oil emulsion droplet technology. The oil droplets may be made using a droplet generator that applies a vacuum to each of the wells (see e.g. Pinheiro et al. Analytical Chemistry 84 (2):1003-11)). Atypically sample may be fractionated into 20,000 or more droplets, and PCR amplification of the template molecules occurs in each individual droplet. Advantageously, ddPCR technology uses reagents and workflows similar to those used for most standard TaqMan probe-based assays.

"Next generation sequencing (NGS)" as used herein shall encompass recently developed technologies for the sequencing of nucleic acids that typically allow much higher throughput than the traditional Sanger approach (see Schuster, Next-generation sequencing transforms today's biology, Nature Methods 5:16-18 (2008); Metzker, Sequencing technologies the next generation. Nat Rev Genet. 2010 January; 11(1):31-46. These platforms can allow sequencing of clonally expanded or non-amplified single molecules of nucleic acid fragments. Certain platforms involve, for example, sequencing by ligation of dye-modified probes (including cyclic ligation and cleavage), pyrosequencing, and single-molecule sequencing. Nucleotide sequence species, amplification nucleic acid species and detectable products generated there from can be analyzed by such sequence analysis platforms. Next-generation sequencing can be used in the methods of the invention, e.g. to quantify unique PCR amplifiable DNA labels in order to assess the formation of bi-component/analyte complexes as described below.

The details of emulsion coupling as a preferred compartmentalized bi-component detection method are described in EP 3224360, which is incorporated by reference herein.

For a ddPCR detection in an emulsion coupling assay the analyte-specific binding components (such as an antibody pair) are labelled by unique PCR amplifiable DNA labels and the thus labeled binding components are added to the sample (or dilutions thereof see e.g. Example 3).

Before an emulsification of the samples, the reaction is highly diluted (~100,000 times) and PCR reagents are added to achieve single-complex separation and PCR amplification per water-in-oil emulsion droplet. ddPCR may be carried out using the standard ddPCR protocol.

The evaluation of the reaction may be based on the partitioning of the labels in a ddPCR reaction using fluorescently tagged PCR products (e.g. using FAM- or VIC-labelled real-time PCR probes). According to the ddPCR, standard evaluation the cluster of droplets may be determined according to the fluorescent signals of the droplets. Herein, the number of labelled binding components (e.g. antibodies) is determined in each reaction (counting all label-positive droplets for a given label, and using the same definition of cluster of droplets for all reactions).

Additionally, the number of the double-colored (having two different binding component labels) is also determined. Without ternary complexes, the partitioning of the labelled antibodies follows Poisson distribution, and results in a calculable number of double-colored droplets (having two binding components in one compartment based upon pure chance). In the case of ternary complexes present in the reaction, the number of the detected double-colored droplets (having additional ternary complexes) is larger than would be expected by Poisson distribution.

On the basis of this measurements the number of complexes can be calculated (see e.g. EP 3224360 or Karakus et al., 2019 for further reference). This results in an absolute (the count of molecules) quantitation of the ternary analyte complexes.

For a next generation sequencing detection in an emulsion coupling assay the binding components may preferably be labelled by unique PCR amplifiable DNA labels, comprising a specific label for binding component (e.g. antibody) and preferably also an individual label for the molecule, i.e. a unique molecular barcode or unique molecular identifier- UMI (see Parekh et al., 2017). The labeled binding components may be added to the samples or dilutions thereof (see Example 3).

After binding of the binding components, e.g. the antibodies, and before the emulsification of samples, the reaction may be highly diluted (e.g. ~100,000 times) and PCR reagents may be added to achieve single-complex separation. PCR amplification can be performed per water-in-oil emulsion droplet. ddPCR may be carried out using ddPCR protocol.

The evaluation of the reaction may be based on the NGS reading of the binding component, e.g. antibody, specific dimerized UMI labels generated according to the standard protocol of emulsion coupling . The number of labelled binding components, e.g. antibodies, may be determined in each reaction by counting all unique UMI labels for a given binding component, e.g. antibody (counting restricted to a given binding component). Possible multiple labelling of the same binding component, e.g. antibody, can be eliminated using their preferentially dimerized sequences (multiple labels per binding component will always result in double UMI label dimers with a given antibody specific label context, as they co-localizing in the same droplet).

Ternary antibody/bi-component complexes are counted based on their dimerized double UMI labeled PCR products (in the context of two different binding component (e.g. antibody) specific labels called heterodimers). A correction for multiple labeling of binding components (e.g. antibodies) may be used following the concept described above, which takes into account double UMI label dimers with a given component (e.g. antibody) specific label.

A further evaluation of the samples can be carried as in case of fluorescently tagged PCR (droplet digital PCR), briefly without ternary complexes, the partitioning of the labelled antibodies follows Poisson distribution, and results in a calculable number of ternary complexes (based on the detection of heterodimers) in droplets (having only two antibodies by chance). In the case of ternary complexes present in the reaction, the number of the detected heterodimers is larger than would be expected by pure Poisson distribution. On the basis of this measurements the number of complexes can be calculated (EP 3224360, (Karakus et al., 2019)). This results in the absolute (the count of molecules) quantitation of the ternary complexes.

The dilutions of the samples can be measured in the same sequencing reaction using samples specific DNA barcodes (e.g. barcoded primers) and as antibodies have distinguishable component (e.g. antibody) specific labels many measurements (using different antibody pairs against different antigens) can be carried out in parallel.

In embodiments of emulsion coupling the binding components may be provided as a library of binding components suitable for the detection of multiple analytes. In these embodiments, each member of the binding component library may be associated with a unique nucleotide sequence, which can be used to identify the binding component.

"Associated" may in the context of emulsion coupling mean that the presence of the binding components in the complex can be detected by the presence of the nucleic acid sequence within the linked sequence generated in the method. The nucleotide sequence may be attached as a label to the binding component, be part of the binding component itself e.g. aptamer, or be present within the binding component e.g. nucleic acid within a phage. For example, each member of the library can be labelled with a unique nucleotide sequence that is a nucleotide sequence is attached to the binding components.

Methods of attaching nucleotides to binding components such as antibody or compounds are known in the art. Alternatively, if the binding component library is a phage display library the unique nucleotide sequence can be the sequence that encodes one or more CDR regions or the displayed binding domain. For example, a display library can be generated by inserting sequences encoding the amino acid sequence to be displayed into a phage at a known location. Universal primers that will amplify the inserted sequences can then be used and thus identify the binding sequence. Alternatively, if the binding component may be an aptamer and the aptamer itself can be the unique nucleotide sequence.

The nucleotide sequence may be an oligonucleotide and may comprise RNA or DNA, single or double stranded. Nucleotides used to label the binding agent or target can preferably be 5- 150 bases in length, for example, 10-40, or 40 -80 bases in length. The nucleotides that form the nucleic acid can be chemically modified to increase the stability of the molecule, to improve its bioavailability or to confer additional activity on it. For example, the pyrimidine bases may be modified at the 6 or 8 positions, and purine bases at the 5 position with CH3 or halogens such as I, Br or Cl. Modifications or pyrimidines bases also include 2 NH3, 0 -CH3, N -CH3 and N2-CH3. Modifications at the 2'position are sugar modifications and include typically a NH2, F or OCH3 group. Modifications can also include 3' and 5' modifications such as capping.

Alternatively, modified nucleotides, such as morpholino nucleotides, locked nucleic acids (LNA) and peptide nucleic acids (PNA) can be used. Morpholino oligonucleotides are assembled from different morpholino subunits, each of which contains one of the four genetic bases (adenine, cytosine, guanine, and thymine) linked to a 6-membered morpholine ring. The subunits are joined by nonionic phosphorodiamidate intersubunit linkages to give a morpholino oligonucleotide. LNA monomers are characterised in that the furanose ring conformation is restricted by a methylene linker that connects the 2'-0 position to the 4'-C position. PNA is an analogue of DNA in which the backbone is a pseudopeptide rather than a sugar.

Each member of a library may be associated with a unique nucleotide sequence, i.e. each member may have a unique detectable, nucleic acid identity labels. Preferably, unique nucleic acid identity labels may be linked. Linked may mean that the linking process has the potential to form random multimer nucleic acid products based on co-localization of these nucleic acid identity labels under suitable assay conditions. Preferably, the multimeric product is a dimer. Suitable assay conditions may include amplifying labels, and specific consensus amplification of the unique sequences produce linkable amplicons. The joining of the linkable amplicons e.g. component specific nucleic acids, form linked identity labels, which encodes the co-localization information of the identity labels. The joining reaction can be amplification based or involve other techniques. Amplification based joining can utilise two or more amplification primer pairs with identical binding abilities, but with complementary 5' tags or dimer linker sequences which result in the formation of polymerase extendable nucleic acid duplexes. The tags or dimer linker sequences mean that the sequence amplified by one primer pair will hybridize to sequences amplified by the second primer pair. The identity labels thereby become linked.

The identity labels i.e. the associated unique nucleotide sequences used in libraries, such as the protein display library and the antibody library, may be different in their biological background, and so the amplification and joining process is based on two different primer pairs, e.g. one primer pair amplifies target sequences such as cDNA based identity labels and the second primer pair amplifies binding agent specific nucleotide sequences used as identity labels. Joining of the different labels makes it possible to link binding agent specific information to target information e.g. proteins encoded by displayed cDNAs.

The method, kit or other computer implemented aspects of the invention may in some embodiments comprise and/or employ one or more conventional computing devices having a processor, an input device such as a keyboard or mouse, memory such as a hard drive and volatile or nonvolatile memory, and computer code (software) for the functioning of the invention.

The components of the computing devices may be conventional, although the device may be custom-configured for each particular implementation. The computer implemented method steps or system may run on any particular architecture, for example, personal/microcomputer, minicomputer, or mainframe systems. Exemplary operating systems include Apple Mac OS X and iOS, Microsoft Windows, and UNIX/Linux; SPARC, POWER and Itanium-based systems;

and z/Architecture. The computer code to perform steps of the method described herein may be written in any programming language or model-based development environment, such as but not limited to Python, C/C++, C#, Objective-C, Java, Basic/VisualBasic, MATLAB, Simulink, StateFlow, Lab View, or assembler. The computer code may comprise subroutines that are written in a proprietary computer language that is specific to the manufacturer of a circuit board, controller, or other computer hardware component used in conjunction with the invention.

The information processed and/or produced by the method, i.e. a digital representations of signals reflecting the formation of ternary bi-component/analyte complexes can employ any kind of file format that is used in the industry. Any suitable computer readable medium may be utilized. The computer-usable or computer-readable medium may be, for example but not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a transmission media such as those supporting the Internet or an intranet, cloud storage or a magnetic storage device.

As used herein the term "comprising" or "comprises" is used in reference to expression cassettes, reporter vectors, and respective component(s) thereof that are open to the inclusion of unspecified elements.

The term "consisting of' refers to expression cassettes, reporter vectors, and respective component(s) thereof as described herein, which are exclusive of any element not recited in that description of the embodiment.

FIGURES

The invention will be explained more in detail by referring to the enclosed drawing figures, in which:

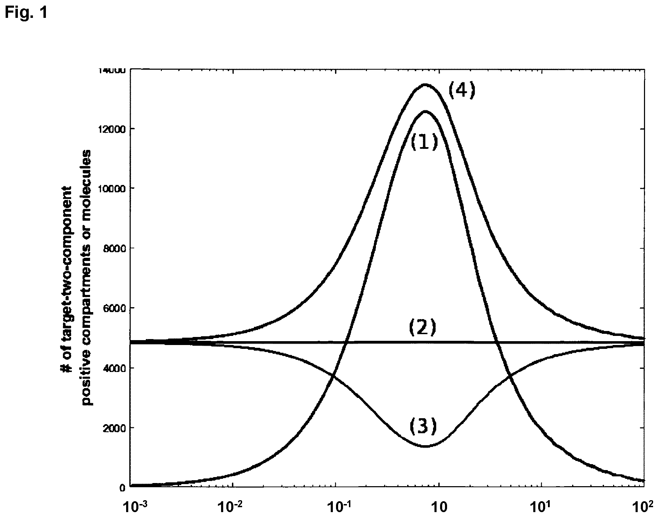

FIG. 1 shows a curve and the basic principle of the mathematical background of the extended dynamic range of the measurement based upon a simulation of a compartmentalized saturable bi-component process. The following parameters have been used: number of compartments = 20 0000, input volume = 20 microL, dilution factor before emulsification = 192000, concentration of target nM - varied, concentration of component A, nM = 1, concentration of component B, nM = 0.3, dissociation constant of component A = 0.1 nM, dissociation constant of component B = 0.1 nM; (1) number of analyte-bi-component, ternary complexes (molecules), (2) - number of double-positive compartments without analyte (Poisson background), (3) - number of double-positive compartments with target (Poisson background), and (4) of sum of (1) and (3).

FIG. 2 show the determination of the dissociation constants of components. Graphical representation of the simulation results. The intersection of the Eq 3 at different antigen (Ag) concentrations (indicated) and at components concentration 1 and 5 nM respectively. Number of droplets: 10000, dilution factor 10000 and the reaction volume is 20 microL. Using these input parameters the chemical balances, the Poisson process is simulated, and number of ternary complexes formed has been determined. The values, using Eq. 3, of the calculated Kds are identical with the values used for the simulation as inputs (see Example 3).

BEST MODE FOR CARRYING OUT THE INVENTION

EXAMPLES

Example 1.

According to the law-of-mass action in solution and law of conservation of mass, the saturable bi-component reactions between two binding components (e.g. antibody) and an analyte/target molecule (e.g. antigen) can be expressed as the following system of equations:

$$kd_1 = \frac{ab_1}{c_1}, kd_2 = \frac{ab_2}{c_2}, kd_1 = \frac{b_1 c_2}{c_{12}}, kd_2 = \frac{b_2 c_1}{c_{12}},$$

$$b_{10} = b_1 + c_1 + c_{12}, b_{20} = b_2 + c_{12} + c_2, a_0 = a + c_1 + c_{12} + c_2 \qquad \text{Eq. 1.}$$

where $b10$, $b20$ are the overall concentrations antibodies (components) with corresponding dissociation constants ($kd 1$, $kd 2$) and $a0$ is the overall antigen concentration, the antibodies and the antigen are either in free $a$, $b 1$, $b 2$ or $c1$,

*c*2,*c* 12 bound state, where *c*12 is the bi-component (ternary) complex (e.g. both antibodies bound) of the antigen (the signal proportional ).

Solving the system of Eq 1. for *a*0 results in two roots:

$$
a0 = \frac{1}{2c_{12}(c_{12} - b_{10})(c_{12} - b_{20})} \left[ c_{12}^4 - c_{12}^3(b_{10} + b_{20} + kd_1 + kd_2) - \right.
$$

$$
c_{12}b_{10}b_{20}(b_{10} + b_{20} + kd_1 + kd_2) + c_{12}^2 \left( 2b_{20}kd_1 + 2b_{10}(b_{20} + kd_2) - \right.
$$

$$
\sqrt{\begin{array}{c}(c_{12}^2 + b_{10}b_{20} - c_{12}(b_{10} + b_{20} + kd_1))^2 - \\ 2c_{12}((c_{12} - b_{10})(c_{12} - b_{20}) + c_{12}kd_1)kd_2 + c_{12}^2kd_2^2 \end{array}} \Bigg) +
$$

$$
b_{10}b_{20} \left( b_{10}b_{20} + \sqrt{\begin{array}{c}(c_{12}^2 + b_{10}b_{20} - c_{12}(b_{10} + b_{20} + kd_1))^2 - \\ 2c_{12}((c_{12} - b_{10})(c_{12} - b_{20}) + c_{12}kd_1)kd_2 + c_{12}^2kd_2^2 \end{array}} \right) \Bigg]
$$

$$
a0 = \frac{1}{2c_{12}(c_{12} - b_{10})(c_{12} - b_{20})} \left[ c_{12}^4 - c_{12}^3(b_{10} + b_{20} + kd_1 + kd_2) - \right.
$$

$$
c_{12}b_{10}b_{20}(b_{10} + b_{20} + kd_1 + kd_2) + c_{12}^2 \left( 2b_{20}kd_1 + 2b_{10}(b_{20} + kd_2) + \right.
$$

$$
\sqrt{\begin{array}{c}(c_{12}^2 + b_{10}b_{20} - c_{12}(b_{10} + b_{20} + kd_1))^2 - \\ 2c_{12}((c_{12} - b_{10})(c_{12} - b_{20}) + c_{12}kd_1)kd_2 + c_{12}^2kd_2^2 \end{array}} \Bigg) +
$$

$$
b_{10}b_{20} \left( b_{10}b_{20} - \sqrt{\begin{array}{c}(c_{12}^2 + b_{10}b_{20} - c_{12}(b_{10} + b_{20} + kd_1))^2 - \\ 2c_{12}((c_{12} - b_{10})(c_{12} - b_{20}) + c_{12}kd_1)kd_2 + c_{12}^2kd_2^2 \end{array}} \right) \Bigg]
$$

Eq 2.

Both of them are real positive roots according to the non-bijective reference curve, i.e. it does not allow for a one-on-one correspondence between the detected signal (c12) and analyte (a0) concentration. Ratios of active/inactive (regarding the ability of binding) antigen and antibodies also can be added to these equations, considering that the overall concentrations of these analytes are not available for the reactions, but only a fraction of them. These ratios can be determined using a concept similar what is described in Example 2.

Example 2.

Simulation of determination of Kds using emulsion coupling (EC) technology

Antigen (Ag) and two different Ag-reactive antibodies reaction (Ab1 = 5 nM and Ab2 = 1 nM) was simulated (according to chemical equilibrium and mass conservation equations (Eq. 1.) with Kd1 = 0.2 nM and Kd2 = 0.1 nM set) in 20 microliter volume. The simulated reaction volume was diluted (1e+5 times) and by Poisson distribution based simulation was used to sort the reactants into 100000 droplets (total volume: 20 microliter). All droplets are classified and counted according to their encapsulated reactants. The kd1 - kd2 functions (Eq. 3) was calculated at three different Ag concentration [a0] with the following inputs: total concentration of Ag [a0], Ab1 [b10], Ab2 [b20] and the simulation determined Ag-Ab1-Ab2 [c12] complex was substituted in equations and the intersection point of functions is determined (see Figure 2).

$$kd_1 = -\frac{-a_0 c_{12}^2 + 2a_0 c_{12} b_{20} - c_{12}^2 b_{20} - a_0 b_{10} b_{20}}{2c_{12} b_{20}} +$$

$$\frac{2c_{12} b_{10} b_{20} - b_{10} b_{20}^2 - c_{12}^2 kd_2 - b_{10} b_{20} kd_2}{2c_{12} b_{20}} -$$

$$\frac{(c_{12}^2 - b_{10} b_{20}) \sqrt{a_0^2 - 2a_0 b_{20} + b_{20}^2 + 2a_0 kd_2 + 2b_{20} kd_2 + kd_2^2}}{2c_{12} b_{20}}$$

$$kd_1 = -\frac{-a_0 c_{12}^2 + 2a_0 c_{12} b_{20} - c_{12}^2 b_{20} - a_0 b_{10} b_{20}}{2c_{12} b_{20}} +$$

$$\frac{2c_{12} b_{10} b_{20} - b_{10} b_{20}^2 - c_{12}^2 kd_2 - b_{10} b_{20} kd_2}{2c_{12} b_{20}} +$$

$$\frac{(c_{12}^2 - b_{10} b_{20}) \sqrt{a_0^2 - 2a_0 b_{20} + b_{20}^2 + 2a_0 kd_2 + 2b_{20} kd_2 + kd_2^2}}{2c_{12} b_{20}}$$

Eq. 3

Eq 3. Ag [a0], Ab1 [b10], Ab2 [b20] and Ag-Ab1-Ab2 [c12] are substituted in equations. In the case of [c12] its value is determined by simulation, the others are input values of the simulation.

Fig. 2 depicts the intersection of the Eq 3 (using equation above) curves at different Ag concentrations. The values of the calculated Kds are identical with the values used for the simulation as inputs (see above).

According to the simulation results, both Kds can be calculated form the initial amount of antigen, and antibody concentrations and the measured concentration of the analyte-bi-component (ternary) complex, by the determination of the intersections of Eq 3. In experiments, it is beneficial if pair-wise intersections are calculated as an approximation of the experimental Kds and the given values are treated by statistical approaches as for instance mixed Gauss distributions or others.

It should be noted, that not all combination of input parameters result in a solvable system, but adequate conditions could always be found, by not only changing the antigen concentration, but changing the concentrations of the antibodies as well. Also only one of the equations (Eq. 3) will result in two positive roots, which in an individual case need to be determined.

Ratios of active/inactive (regarding the ability of binding) antigen and antibodies also can be added to these equations, considering that the overall concentrations of these analytes are not available for the reactions, but only a fraction of them. These ratios can be determined using more dilutions extending the concept described in Example 2.

Example 3.

Disclosed is also an exemplary measurement of an analyte concentration useful for understanding the invention.

A sample containing an unknown concentration of a known analyte is provided. As an exemplary case the analyte is a protein, also as an example it is HER2, an EGFR (epidermal growth factor receptor) receptor variant. To detect the analyte using a bi-component method, Alphascreen is applied. Alphascreen is a prototype bi-component detection method, where the signal generation relies on the proximity of "donor" and "acceptor" beads coated with a layer of hydrogel providing functional groups for bioconjugation. When a biological interaction between molecules brings the beads into proximity, a cascade of chemical reactions is initiated to produce a greatly amplified signal. Upon laser excitation, a photosensitizer in the "donor" bead converts ambient oxygen to a more excited singlet state. The singlet state oxygen molecules diffuse across to react with a chemiluminescer in the "acceptor" bead that further activates fluorophores contained within the same bead. The fluorophores subsequently emit light at 520-620 nm. The functional groups, which are involved in the biological interaction is provided by two antibodies, as an exemplary figure ErbB2/Her2 Antibody Pair (Novus Biologicals DP0061), the antibodies are conjugated to the surface of the donor and acceptor beads, respectively. Upon contact of the donor and acceptor beads to the sample, the antibodies will bind to the HER2 protein forming a ternary complex, which holds the donor and acceptor beads in close proximity resulting in a detectable fluorescent signal according to the principle described above.

To measure the unknown concentration of a HER2 protein analyte a comparison can be made to predetermined signals of a series of different known concentrations of HER2 proteins. Such a reference curve for a bi-component assay is characterized by a certain signal value that has a correspondence to two analyte concentration values in most cases, e.g. a non-bijective reference curve. Hence, the curve does not allow for a one-on-one correspondence between the analyte concentration and the detected signal. To determine the concentration of HER2 one or more defined factor dilutions are made from the sample of unknown amount of HER2 and the signals a determined. Each signal of the dilutions has two corresponding concentrations if compared to the reference curve. As the ratio of the deduced concentrations and their defined dilution factor must be equal, the concentration of the HER2 sample can be determined. Mathematically $f(x) = s1$ where $f(x)$ the reference curve function, which a function of concentration $x$ and $s1$ is the measured signal. $F(x) = s1$ can be satisfied by two concentrations $x1$ or $x2$. However, $x$ can be determined if $f(x/c) = s2$ is determined, where $c$ a defined dilution factor and $s2$ the measured signal; similarly, as above, $f(x/c) = s2$ can be satisfied by $x3$ or $x4$. As $x1/x3$ or $x1/x4$ or $x2/x3$ or $x2/x4$ must be $c$ and it can be proved that only one or two of them can be equal to $c$ and if two of them are equal they can be satisfied at the same $x$ only, thus the ambiguity can be solved. For example if $s1=1000$ fluorescent unit (FU), it corresponds in hypothetical reference curve to 100 nM ($x1$) and 5 nM ($x2$) HER2 protein, diluting the original HER2 sample by a factor of 10 results in 2000 (FU) signal, which corresponds in a hypothetical reference curve to 90 nM ($x3$) and 10 nM ($x4$) HER2 proteins. $x1/x3=100/90$ or $x1/x4=100/10$ or $x2/x3=5/90$ or $x2/x4=5/10$ as only $x1/x4=c$ the results is $x = x1 = 100$ nM.

Example 4.

Disclosed is also an exemplary measurement of an analyte concentration using a compartmentalized bi-component method useful for understanding the invention.

A sample containing an unknown concentration of a known analyte is provided. As an exemplary case, the analyte is a protein, also as an example it is HER2, an EGFR receptor variant, the ErbB2/Her2 Antibody Pair (Novus Biologicals DP0061) is used as detection reagents, i.e. as analyte-specific binding components. The compartmentalized bi-component method is emulsion coupling (EP 3224360, Karakus et al.). Emulsion coupling is a digital assay concept based on the detection of double-labelled (bi-component), individual ternary molecular complexes in emulsion, which are identified, for example, by ddPCR. For ddPCR detection, ErbB2/Her2 Antibody Pair (Novus Biologicals DP0061) are labelled by unique PCR amplifiable DNA labels. The sample is diluted by defined factors and the labeled antibodies are added to the samples, as in Example 3. Before the emulsification of samples, the reaction is highly diluted (~100,000 times) and PCR reagents are added to achieve single-complex separation and PCR amplification per water-in-oil emulsion droplet. ddPCR is carried out using the standard ddPCR protocol (Biorad QX200 ddPCR).

The evaluation of the reaction is based on the partitioning of the labels in the ddPCR reactions using fluorescently tagged PCR products (using FAM- or VIC-labelled real-time PCR probes). According to the ddPCR standard evaluation the cluster of droplets are determined according to the fluorescent signals of the droplets, the number of labelled antibodies are determined in each reaction (counting all label-positive droplets for a given label, and using the same definition of cluster of droplets for all reactions). Additionally the number of the double-colored (having two different antibody labels) is also determined. Without ternary complexes (HER2 protein with two antibodies bound), the partitioning of the labelled antibodies follows a Poisson distribution, and results in a calculable number of double-colored droplets (having two antibodies based upon pure chance). In the case of ternary complexes present in the reaction, the number of the detected double-colored droplets (having additional ternary complexes) is larger than would be expected by Poisson distribution. On the basis of this measurements the number of complexes can be calculated (see EP 3224360, Karakus et al., 2019 for further reference). This results in the absolute (the count of molecules) quantitation of the ternary analyte complexes. According to Eq. 1. with known dissociation constants and active fraction of the applied antibodies the original absolute concentration of the analyte (HER2) also can be calculated. However Eq 1. has two roots, only one of them is the concentration. Following a similar calculation as in Example 3 given, the correct concentration of the unknown HER2 sample can be determined (see Example 3 for a numerical example).

Example 5.

An exemplary measurement of dissociation constants of antibodies in a non-compartmentalized bi-component assay

A sample containing a known concentration of a known analyte is provided. As an exemplary case, the analyte is a protein, also as an example it is HER2, an EGFR receptor variant, ErbB2/Her2 Antibody Pair (Novus Biologicals DP0061) is used as an exemplary reagent. The bi-component method is Alphascreen as in Example 3 using dilutions and reference curve described in Example 3. To determine the relationship of the Alphascreen signal and the corresponding concentration of the ternary complexes, one can use a method described in Example 4 parallel to the Alphascreen reference curve measurements. This results in the absolute (the count of molecules) quantitation of the ternary complexes related to the Alphascreen signals. To calculate the dissociation constants (Kd) of the antibodies Eq 3. are applied, having more than one analyte dilutions with known amount of analyte and the determined amount of ternary complex - the latter is determined using the determined correspondence between the concentration of the ternary complexes and the Alphascreen signal. The two Kds can be calculated according to the Example 2. Mathematically $f(kd1,c12,a0) = kd2$ where kd1 and kd2 are dissociation constants and c12 is the measured concentration of ternary complex of the analyte and a is the concentration of the analyte, is applied. As different dilutions of the analyte corresponds to different concentration of the c12, the f function has different forms. However as the Kds are material constants, consequently all forms of the f function must be satisfied by a certain pair of values of Kds, which can be determined, see Example 2.

Example 6.

An exemplary measurement of dissociation constants of antibodies in a compartmentalized bi-component assay

A sample containing a known concentration of a known analyte is provided. As an exemplary case, the analyte is a protein, also as an example it is HER2, an EGFR receptor variant, ErbB2/Her2 Antibody Pair (Novus Biologicals DP0061) is used as exemplary detection reagents, i.e. analyte-specific binding components. The compartmentalized bi-component method is emulsion coupling (EP 3224360, (Karakus et al., 2019)) using Biorad QX200 ddPCR as detection device as in Example 4 using dilutions and the number of ternary complexes can be calculated (EP 3224360, Karakus et al., 2019) as in Example 4. This results in the absolute (the count of molecules) quantitation of the ternary complexes. To calculate the dissociation constants of the antibodies Eq 3. are applied, having more than one analyte dilutions with known amount of analyte, antibodies and determined amount of ternary complex, the two Kd can be calculated according to the Example 2. Mathematically $f(kd1,c12,a0) = kd2$ is applied, where kd1 and kd2 are dissociation constants and ac is the measured concentration of ternary complex of the analyte and a is the concentration of the analyte. As different dilutions corresponds to different (concentration) values of the 'ac', the f function has different forms. However, since the Kds are material constants, consequently all forms of the f function must be satisfied by a certain pair of values of Kds, which can be determined, see Example 2.

Example 7.

Disclosed is also an exemplary measurement of an analyte concentration using a compartmentalized bi-component method using next-generation sequencing useful for understanding the invention.

A sample containing an unknown concentration of a known analyte is provided. As an exemplary case, the analyte is a protein, also as an example it is HER2, an EGFR receptor variant, ErbB2/Her2 Antibody Pair (Novus Biologicals DP0061) are used as detection reagents, i.e. analyte-specific binding components. The compartmentalized bi-component method is emulsion coupling (EP 3224360, Karakus et al., 2019)). Emulsion coupling is a digital assay concept based on the detection of double-labelled (ternary), individual molecular complexes in emulsion, which are identified, for example, by next-generation sequencing (NGS). For NGS detection the ErbB2/Her2 Antibody Pair (Novus Biologicals DP0061) are labelled by unique PCR amplifiable DNA labels (antibody specific label), which also has the individual label molecule unique molecular barcode (UMI) as well (Parekh et al., 2017). Defined analyte dilutions are made and the labeled antibodies are added to the samples, as in Example 3. After antibody binding and before the emulsification of samples, the reaction is highly diluted (~100,000 times) to achieve single-complex separation, PCR reagents are added and PCR amplification per water-in-oil emulsion droplet like in ddPCR is carried out using ddPCR protocol (Biorad QX200 ddPCR).

The evaluation of the reaction is based on the NGS reading of the antibody specific dimerized UMI labels generated according to emulsion coupling concept (EP 3224360). The number of labelled antibodies is determined in each reaction by counting all unique UMI labels for a given antibody (counting restricted to a given antibody specific label context), and the possible multiple labels of the same antibody was eliminated using their preferentially dimerized sequences (multiple labels per antibody always have double UMI label dimers with the same antibody specific label context, as they co-localizing in the same droplet). The ternary complexes are counted on the basis of their dimerized double UMI labeled PCR products (two different antibody specific labels called heterodimers) with correction according to the antibody multiple labels (following the concept presented above to eliminate the effect of the multiple labels per antibody). The further evaluation of the samples is carried out according to Example 4, briefly without ternary complexes, the partitioning of the labelled antibodies follows Poisson distribution, and results in a calculable number of ternary complexes (based on the detection of heterodimers) in droplets (having only two antibodies by chance). In the case of ternary complexes present in the reaction, the number of the detected heterodimers is larger than would be expected by pure Poisson distribution. On the basis of this measurements the number of complexes can be calculated (EP 3224360). This results in the absolute (the count of molecules) quantitation of the ternary complexes. According to Eq. 1-2 with known dissociation constants and active fraction (latter is optional) of the applied antibodies the original absolute concentration of the analyte (HER2) also can be calculated. However, Eq 1-2 have two roots, only one of them always is real. As described in Example 3, using dilutions the correct concentration of the unknown HER2 sample can be determined (see Example 3 for a numerical example). The dilutions of the samples can be measured in the same sequencing reaction using samples specific DNA barcodes (e.g. barcoded primers).

Example 8.

An exemplary measurement of dissociation constants of antibodies in a compartmentalized bi-component assay using next-generation sequencing

A sample containing a known concentration of a known analyte is provided. As an exemplary case, the analyte is a protein, also as an example it is HER2, an EGFR receptor variant, ErbB2/Her2 Antibody Pair (Novus Biologicals DP0061) labeled with antibody specific UMI labels. The compartmentalized bi-component method was emulsion coupling (EP 3224360, ) using NGS as the detection method as in Example 7 and using dilutions and Eq1-2. the number of ternary complexes can be calculated (EP 3224360). This results in the absolute (the count of molecules) quantitation of the ternary complexes. To calculate the dissociation constants (Kd) of the antibodies Eq. 3. is applied, having more than one analyte dilutions (these can be partially the same dilutions as above) with known amount of analyte and determined amount of ternary complex the two Kd can be calculated according to the Example 2. Mathematically $f(kd1, c12, a0) = kd2$ where $kd1$ and $kd2$ are dissociation constants and $c12$ is the measured concentration of ternary complex of the analyte and $a0$ is the concentration of the analyte, is applied. As different dilutions corresponds to different (concentration) values of the $c12$, the f function has different forms, however as the Kds are material constants, consequently all forms of the f function must be satisfied by a certain pair of values of Kds, which can be determined, see Example 2. The dilutions of the samples can be measured in the same sequencing reaction using samples specific DNA barcodes (e.g. barcoded primers).

Ratios of active/inactive (regarding the ability of binding) antigen and antibodies also can be added to these equations, considering that the overall concentrations of these analytes are not available for the reactions, but only a fraction of them. These ratios can be determined using more dilutions extending the concept described in Example 8.

REFERENCES

Binder H. et al. (2008) "Hook"-calibration of GeneChip-microarrays: Theory and algorithm, Algorithms for molecular biology, Biomed Central LTD, LO, vol. 3, no. 1, page 12, 29 August 2008.

Douglass E. F. ETAL: "A Comprehensive Mathematical Model for Three-Body Binding Equilibria", Journal of the American Chemical Society, vol. 135, no. 16, 16 April 2013.

Friguet, B., Chaffotte, A. F., Djavadi-Ohaniance, L., & Goldberg, M. E. (1985). Measurements of the true affinity constant in solution of antigen-antibody complexes by enzyme-linked immunosorbent assay. Journal of Immunological Methods, 77(2), 305-319. Retrieved from http://www.ncbi.nlm.nih.gov/pubmed/3981007

Karakus, U., Thamamongood, T., Ciminski, K., Ran, W., Günther, S. C., Pohl, M. O., ... Stertz, S. (2019). MHC class II proteins mediate cross-species entry of bat influenza viruses. Nature 567(7746) https://doi.org/10.1038/s41586-019-0955-3

Morell, M., Ventura, S., & Avilés, F. X. (2009). Protein complementation assays: Approaches for the in vivo analysis of protein interactions. FEBS Letters, 583(11), 1684-1691. https://doi.org/10.1016/j.febslet.2009.03.002

Parekh, S., Ziegenhain, C., Vieth, B., Enard, W., & Hellmann, I. (2017). zUMIs: A fast and flexible pipeline to process RNA sequencing data with UMIs. BioRxiv, 2-7. https://doi.org/10.1101/153940

Pfleger, K. D. G., Seeber, R. M., & Eidne, K.A. (2006). Bioluminescence resonance energy transfer (BRET) for the real-time detection of protein-protein interactions. Nature Protocols, 1(1), 337-345. https://doi.org/10.1038/nprot.2006.52

Quan, P. L., Sauzade, M., & Brouzes, E. (2018). DPCR: A technology review. Sensors (Switzerland), 18(4). https://doi.org/10.3390/s18041271

Rey E. G. ETAL (2017): "Mitigating the Hook Effect in Lateral Flow Sandwich Immunoassays Using Real-Time Reaction Kinetics", ANALYTICAL CHEMISTRY, vol. 89, no. 9, pages 5095-5100, XP055566188.

Rossant C. J. et al. (2015): "Versatility of homogeneous time-resolved fluorescence resonance energy transfer assays for biologic drug discovery", Journal of Biomolecular Screening Society for Laboratory Automation and Screening.

Xiong Y. et al. (2017): "Development of a novel immunoassay to detect interactions with the transactivation domain of p53: application to screening of new drugs", SCIENTIFIC REPORTS, vol. 7, no. 1.

Smith, A. M., Heisler, L. E., Mellor, J., Kaper, F., Thompson, M. J., Chee, M., ... Nislow, C. (2009). Quantitative phenotyping via deep barcode sequencing. Genome Research, 19(10), 1836-1842. https://doi.org/10.1101/gr.093955.109

Söderberg, O., Gullberg, M., Jarvius, M., Ridderstråle, K., Leuchowius, K.-J., Jarvius, J., ... Landegren, U. (2006). Direct observation of individual endogenous protein complexes in situ by proximity ligation. Nature Methods, 3(12), 995-1000. https://doi.org/10.1038/nmeth947

Taouji, S., Dahan, S., Bosse, R., & Chevet, E. (2009). Current Screens Based on the AlphaScreen™ Technology for Deciphering Cell Signalling Pathways. Current Genomics, 10(2), 93-101. https://doi.org/10.2174/138920209787847041

Wu et al. (2018). Förster resonance energy transfer immunoassays using engineered proteins for breast cancer biomarker detection. Ph.D. Thesis. Université Paris-Saclay.

Zorba A. et al.: "Delineating the role of cooperativity in the design of potent PROTACs for BTK", Proceedings of the national academy of sciences, vol. 115, no. 31, 16 July 2018.

## Claims

1. A method for determining the dissociation constants of analyte-specific binding components with an analyte in a sample of known concentration in a bi-component detection method comprising:

   a. providing two non-immobilized analyte-specific binding components at known concentrations for conducting a bi-component detection method in a solution comprising bringing said two analyte-specific binding components in contact with the analyte to produce a signal that depends on the concentration of bi-component/analyte complexes formed in said solution,
   b. providing a dissociation constant relationship for said bi-component detection method which is a mathematical function for the relationship between the dissociation constants of the two analyte-specific binding components with the analyte (kd1 and kd2, respectively) in dependence of said signal reflecting the concentration of bi-component/analyte complexes and the concentration of analyte and analyte-specific binding components,
   c. preparing one or more dilutions of said sample and/or said analyte-specific binding components,
   d. applying the bi-component detection method to the sample and the one or more dilutions,
   e. determining the dissociations constants kd1 and kd2 of said two analyte-specific binding components using said signal detected in the sample and in the one or more dilutions as a constraining input for a mathematical fit for said dissociation constant relationship at different analyte concentration and/or analyte-specific binding components concentrations.

2. The method according to claim 1, wherein said dissociation constant relationships are calculated analytically by solving chemical balance, and mass conservation equations.

3. The method according to any of the preceding claims, wherein the analyte is selected from a group consisting of proteins, peptides, nucleic acid segments, carbohydrates, lipids, antibodies (monoclonal or polyclonal), antigens, oligonucleotides, specific receptor proteins, ligands, molecules, cells, microorganisms as well as fragments products or combinations of thereof and/or wherein the sample comprise two or more different types of analytes.

4. The method according to any of the preceding claims, wherein the two analyte-specific binding components are selected from a group consisting of nucleic acids, preferably RNA and/or DNA oligonucleotides, antibodies, peptides, proteins, aptamers, molecularly-imprinted polymers, cells or combinations of thereof and/or wherein two or more pairs of different kind of analyte-specific binding components are used.

5. The method according to any of the preceding claims, wherein the bi-component method comprises employing a proximity-based assay to produce the bi-component/analyte complexes concentration dependent signal, wherein the proximity-based assay uses two analyte-specific binding components that produce a detectable signal in dependence of their proximity.

6. The method according to any of the preceding claims, wherein the bi-component method comprises employing a resonance energy transfer assay, preferably a Förster resonance energy transfer (FRET) assay or a bioluminescence resonance energy transfer (BRET) assay, a protein complementation assay (PCA), Alphascreen or a DNA labeled proximity assay, preferably a proximity ligation assay (PLA) or a proximity extension assay (PEA).

7. The method according to any of the preceding claims, wherein the bi-component detection method comprises employing a compartmentalized assay to produce a bi-component/analyte complexes concentration depended signal, wherein the signal reflects the presence of said two analyte-specific binding components in a single compartment wherein the compartmentalized assay preferably employs an emulsion droplet method, wherein each droplet in the emulsion represents a separate compartment, more preferably wherein the compartmentalized assay is emulsion coupling.

8. The method according to any one of the preceding claims, wherein multiple analytes are determined in parallel.

9. The method according to any one of the preceding claims, wherein the bi-component detection method comprises employing an absolute molecular count based analytical method and/or wherein the bi-component detection method comprises employing a droplet digital PCR assay.

10. The method according to any one of the preceding claims, wherein the bi-component detection method comprises using analyte-specific binding components associated with unique amplifiable nucleic acid labels and employs a compartmentalized assay, wherein a nucleic acid amplification is performed for each compartment using fluorescently tagged amplification products.

11. The method according to any one of the preceding claims, wherein the bi-component detection method comprises using multiple analyte-specific binding components comprising nucleic acid molecule-identifying - unique molecular identifier - barcodes and employs a compartmentalized assay, wherein an nucleic acid amplification is performed for each compartment producing linked molecule-identifying - unique molecular identifier - nucleic acid barcodes, the compartments are reunited in a common pool and a parallel nucleic acid sequencing technique is used to produce the bi-component/analyte complexes concentration depended signal.

12. The method according to any one of the preceding claims 10 or 11, wherein the parallel nucleic acid sequencing technique is a next generation sequencing technique (NGS).

13. A Kit for determining dissociation constants in a bi-component detection method comprising:

    a. reaction buffer for two or more analyte-specific binding components at known concentrations for conducting a bi-component detection method comprising bringing said two analyte-specific binding components in contact with a solution containing an analyte to produce a signal that depends on the concentration of bi-component/analyte complexes formed in said solution,
    b. reference data on a dissociation constant relationship for said bi-component detection method which is a mathematical function for the relationship between kd1 and kd2 in dependence of said signal reflecting the concentration of bi-component/analyte complexes and the concentration of analytes and/or analyte-specific binding components,
    c. optionally instructions for preparing one or more dilutions of said sample and applying the bi-component detection method to the sample and the one or more dilutions using defined dilutions factors,
    d. a computer program that, when executed on a computer, is configured for performing the computational steps of determining the dissociations constants kd1 and kd2 of said two analyte-specific binding component using said signal detected in the sample and in the one or more dilutions as a constraining input for a mathematical fit for said dissociation constant relationship at different analyte and/or analyte-specific binding components concentrations.

**Patentansprüche**

1. Verfahren zum Bestimmen der Dissoziationskonstanten analytspezifischer Bindungskomponenten mit einem Analyten in einer Probe bekannter Konzentration in einem Zweikomponenten-Nachweisverfahren, umfassend:

   a. Bereitstellen von zwei nicht immobilisierten analytspezifischen Bindungskomponenten in bekannten Konzentrationen zum Durchführen eines Zweikomponenten-Nachweisverfahrens in einer Lösung, umfassend Inkontaktbringen der zwei analytspezifischen Bindungskomponenten mit dem Analyten, um ein Signal zu erzeugen, das von der Konzentration von Zweikomponenten-/Analyt-Komplexen abhängt, die in der Lösung ausgebildet sind,
   b. Bereitstellen einer Dissoziationskonstantenbeziehung für das Zweikomponenten-Nachweisverfahren, welche eine mathematische Funktion für die Beziehung zwischen den Dissoziationskonstanten der zwei analytspezifischen Bindungskomponenten mit dem Analyten (kd1 bzw. kd2) in Abhängigkeit von dem Signal ist, das die Konzentration von Zweikomponenten-/Analyt-Komplexen und die Konzentration von Analyt und analytspezifischen Bindungskomponenten widerspiegelt,
   c. Herstellen einer oder mehrerer Verdünnungen der Probe und/oder der analytspezifischen Bindungskomponenten,
   d. Anwenden des Zweikomponenten-Nachweisverfahrens auf die Probe und die eine oder mehreren Verdünnungen,
   e. Bestimmen der Dissoziationskonstanten kd1 und kd2 der zwei analytspezifischen Bindungskomponenten unter Verwendung des in der Probe und in der einen oder den mehreren Verdünnungen nachgewiesenen Signals als eine einschränkende Eingabe für eine mathematische Anpassung der Dissoziationskonstantenbeziehung bei unterschiedlichen Analytkonzentrationen und/oder Konzentrationen analytspezifischer Bindungskomponenten.

2. Verfahren nach Anspruch 1, wobei die Dissoziationskonstantenbeziehungen durch Lösen von Gleichungen für das chemische Gleichgewicht und die Massenerhaltung analytisch berechnet werden.

3. Verfahren nach einem der vorhergehenden Ansprüche, wobei der Analyt aus einer Gruppe bestehend aus Proteinen, Peptiden, Nukleinsäuresegmenten, Kohlenhydraten, Lipiden, Antikörpern (monoklonal oder polyklonal), Antigenen, Oligonukleotiden, spezifischen Rezeptorproteinen, Liganden, Molekülen, Zellen, Mikroorganismen sowie Fragmenten, Produkten oder Kombinationen davon ausgewählt ist und/oder wobei die Probe zwei oder mehr verschiedene Arten von Analyten umfasst.

4. Verfahren nach einem der vorhergehenden Ansprüche, wobei die zwei analytspezifischen Bindungskomponenten aus einer Gruppe bestehend aus Nukleinsäuren, vorzugsweise RNA- und/oder DNA-Oligonukleotiden, Antikörpern, Peptiden, Proteinen, Aptameren, molekular geprägten Polymeren, Zellen oder Kombinationen davon ausgewählt sind und/oder wobei zwei oder mehrere Paare verschiedener Arten von analytspezifischen Bindungskomponenten verwendet werden.

5. Verfahren nach einem der vorhergehenden Ansprüche, wobei das Zweikomponentenverfahren Einsetzen eines Proximitybasierten Assays umfasst, um das konzentrationsabhängige Signal der Zweikomponenten-/Analyt-Komplexe zu erzeugen, wobei der Proximity-basierte Assay zwei analytspezifische Bindungskomponenten verwendet, die in Abhängigkeit von ihrer Nähe ein nachweisbares Signal erzeugen.

6. Verfahren nach einem der vorhergehenden Ansprüche, wobei das Zweikomponentenverfahren Einsetzen eines Resonanzenergietransfer-Assays, vorzugsweise eines Förster-Resonanzenergietransfer(FRET)-Assays oder eines Biolumineszenz-Resonanzenergietransfer (BRET)-Assays, eines Proteinkomplementierungs-Assays (PCA), Alphascreen oder eines DNA-markierten Proximity Assays, vorzugsweise eines Proximity Ligation Assays (PLA) oder eines Proximity Extension Assays (PEA), umfasst.

7. Verfahren nach einem der vorhergehenden Ansprüche, wobei das Zweikomponenten-Nachweisverfahren Einsetzen eines kompartimentierten Assays umfasst, um ein konzentrationsabhängiges Signal von Zweikomponenten-/Analyt-Komplexen zu erzeugen, wobei das Signal das Vorhandensein der zwei analytspezifischen Bindungskomponenten in einem einzelnen Kompartiment widerspiegelt, wobei der kompartimentierte Assay vorzugsweise ein Emulsionströpfchenverfahren einsetzt, wobei jedes Tröpfchen in der Emulsion ein separates Kompartiment darstellt, wobei der kompartimentierte Assay vorzugsweise eine Emulsionskopplung ist.

8. Verfahren nach einem der vorhergehenden Ansprüche, wobei mehrere Analyten parallel bestimmt werden.

9. Verfahren nach einem der vorhergehenden Ansprüche, wobei das Zweikomponenten-Nachweisverfahren Einsetzen eines auf der absoluten Molekülzahl basierten Analyseverfahrens umfasst und/oder wobei das Zweikomponenten-Nachweisverfahren Einsetzen eines digitalen Tröpfchen-PCR-Assays umfasst.

10. Verfahren nach einem der vorhergehenden Ansprüche, wobei das Zweikomponenten-Nachweisverfahren Verwenden von analytspezifischen Bindungskomponenten umfasst, die eindeutigen amplifizierbaren Nukleinsäuremarkierungen zugeordnet sind, und einen kompartimentierten Assay einsetzt, wobei eine Nukleinsäureamplifikation für jedes Kompartiment unter Verwendung von mit einem Fluoreszenz-Tag versehenen Amplifikationsprodukten durchgeführt wird.

11. Verfahren nach einem der vorhergehenden Ansprüche, wobei das Zweikomponenten-Nachweisverfahren Verwenden mehrerer analytspezifischer Bindungskomponenten umfasst, die Strichcodes zur Identifizierung von Nukleinsäuremolekülen - eindeutige Molekülkennungen - umfassen, und einen kompartimentierten Assay einsetzt, wobei eine Nukleinsäureamplifikation für jedes Kompartiment durchgeführt wird, das verknüpfte Nukleinsäure-Strichcodes zur Identifizierung von Molekülen - eindeutige Molekülkennungen - erzeugt, wobei die Kompartimente in einem gemeinsamen Pool wieder vereint werden und eine parallele Nukleinsäuresequenzierungstechnik verwendet wird, um das konzentrationsabhängige Signal von Zweikomponenten-/Analyt-Komplexen zu erzeugen.

12. Verfahren nach einem der vorhergehenden Ansprüche 10 oder 11, wobei die parallele Nukleinsäuresequenzierungstechnik eine Sequenzierungstechnik der nächsten Generation (next generation sequencing - NGS) ist.

13. Kit zum Bestimmen von Dissoziationskonstanten in einem Zweikomponenten-Nachweisverfahren, umfassend:

a. Reaktionspuffer für zwei oder mehr analytspezifische Bindungskomponenten in bekannten Konzentrationen zum Durchführen eines Zweikomponenten-Nachweisverfahrens, umfassend Inkontaktbringen der zwei analytspezifischen Bindungskomponenten mit einer einen Analyten enthaltenden Lösung, um ein Signal zu erzeugen, das von der Konzentration von Zweikomponenten-/Analyt-Komplexen abhängt, die in der Lösung ausgebildet sind,

b. Referenzdaten zu einer Dissoziationskonstantenbeziehung für das Zweikomponenten-Nachweisverfahren, welche eine mathematische Funktion für die Beziehung zwischen kd1 und kd2 in Abhängigkeit von dem Signal ist, das die Konzentration von Zweikomponenten-/Analyt-Komplexen und die Konzentration von Analyten und/oder analytspezifischen Bindungskomponenten widerspiegelt,

c. optional Anweisungen zum Herstellen einer oder mehrerer Verdünnungen der Probe und zum Anwenden des Zweikomponenten-Nachweisverfahrens auf die Probe und die eine oder mehreren Verdünnungen unter Verwendung definierter Verdünnungsfaktoren,

d. ein Computerprogramm, das bei Ausführung auf einem Computer zum Durchführen der Rechenschritte zum Bestimmen der Dissoziationskonstanten kd1 und kd2 der zwei analytspezifischen Bindungskomponenten unter Verwendung des in der Probe und in der einen oder den mehreren Verdünnungen nachgewiesenen Signals als eine einschränkende Eingabe für eine mathematische Anpassung der Dissoziationskonstantenbeziehung bei unterschiedlichen Konzentrationen von Analyten und/oder analytspezifischen Bindungskomponenten konfiguriert ist.

## Revendications

1. Procédé pour déterminer les constantes de dissociation de composants de liaison spécifiques à un analyte avec un analyte dans un échantillon de concentration connue dans un procédé de détection à deux composants comprenant :

a. la fourniture de deux composants de liaison spécifiques à un analyte non immobilisés à des concentrations connues pour mettre en œuvre un procédé de détection à deux composants dans une solution comprenant la mise en contact desdits deux composants de liaison spécifiques à l'analyte avec l'analyte pour produire un signal qui dépend de la concentration de complexes bicomposant/analyte formés dans ladite solution,

b. la fourniture d'une relation de constantes de dissociation pour ledit procédé de détection à deux composants qui est une fonction mathématique pour la relation entre les constantes de dissociation des deux composants de liaison spécifiques à l'analyte avec l'analyte (kd1 et kd2, respectivement) en fonction dudit signal reflétant la concentration des complexes bicomposant/analyte et la concentration de l'analyte et des composants de liaison spécifiques à l'analyte,

c. la préparation d'une ou de plusieurs dilutions dudit échantillon et/ou desdits composants de liaison spécifiques à l'analyte,

d. l'application du procédé de détection à deux composants à l'échantillon et à la ou aux dilutions,

e. la détermination des constantes de dissociation kd1 et kd2 desdits deux composants de liaison spécifiques à l'analyte en utilisant ledit signal détecté dans l'échantillon et dans la ou les dilutions en tant qu'entrée contraignante pour un ajustement mathématique pour ladite relation de constantes de dissociation à une concentration d'analyte et/ou à des concentrations de composants de liaison spécifique à l'analyte différentes.

**2.** Procédé selon la revendication 1, dans lequel lesdites relations de constantes de dissociation sont calculées analytiquement en résolvant des équations d'équilibre chimique et de conservation de masse.

**3.** Procédé selon l'une quelconque des revendications précédentes, dans lequel l'analyte est choisi dans un groupe constitué de protéines, de peptides, de segments d'acide nucléique, de glucides, de lipides, d'anticorps (monoclonaux ou polyclonaux), d'antigènes, d'oligonucléotides, de protéines réceptrices spécifiques, de ligands, de molécules, de cellules, de micro-organismes ainsi que de produits de fragments, ou de combinaisons de ceux-ci et/ou dans lequel l'échantillon comprend deux ou plusieurs types différents d'analytes.

**4.** Procédé selon l'une quelconque des revendications précédentes, dans lequel les deux composants de liaison spécifiques à l'analyte sont choisis dans un groupe constitué d'acides nucléiques, de préférence d'oligonucléotides d'ARN et/ou d'ADN, d'anticorps, de peptides, de protéines, d'aptamères, de polymères à empreinte moléculaire, de cellules ou de combinaisons de ceux-ci et/ou dans lequel deux paires ou plus de types différents de composants de liaison spécifiques à l'analyte sont utilisées.

**5.** Procédé selon l'une quelconque des revendications précédentes, dans lequel le procédé à deux composants comprend l'utilisation d'un test basé sur la proximité pour produire le signal dépendant de la concentration des complexes bicomposant/analyte, dans lequel le test basé sur la proximité utilise deux composants de liaison spécifiques à l'analyte qui produisent un signal détectable en fonction de leur proximité.

**6.** Procédé selon l'une quelconque des revendications précédentes, dans lequel le procédé à deux composants comprend l'utilisation d'un test de transfert d'énergie par résonance, de préférence un test de transfert d'énergie par résonance de Förster (FRET) ou un test de transfert d'énergie par résonance par bioluminescence (BRET), un test de complémentation protéique (PCA), un test Alphascreen ou un test de proximité marqué à l'ADN, de préférence un test de ligature de proximité (PLA) ou un test d'extension de proximité (PEA).

**7.** Procédé selon l'une quelconque des revendications précédentes, dans lequel le procédé de détection à deux composants comprend l'utilisation d'un essai compartimenté pour produire un signal dépendant de la concentration des complexes bicomposant/analyte, dans lequel le signal reflète la présence desdits deux composants de liaison spécifiques à l'analyte dans un seul compartiment dans lequel l'essai compartimenté utilise de préférence un procédé de gouttelettes d'émulsion, dans lequel chaque gouttelette dans l'émulsion représente un compartiment séparé, plus préférablement dans lequel l'essai compartimenté est un couplage d'émulsion.

**8.** Procédé selon l'une quelconque des revendications précédentes, dans lequel plusieurs analytes sont déterminés en parallèle.

**9.** Procédé selon l'une quelconque des revendications précédentes, dans lequel le procédé de détection à deux composants comprend l'utilisation d'un procédé analytique basé sur le comptage moléculaire absolu et/ou dans lequel le procédé de détection à deux composants comprend l'utilisation d'un test PCR numérique de gouttelettes.

**10.** Procédé selon l'une quelconque des revendications précédentes, dans lequel le procédé de détection à deux composants comprend l'utilisation de composants de liaison spécifiques à l'analyte associés à des marqueurs d'acide nucléique amplifiables uniques et utilise un essai compartimenté, dans lequel une amplification d'acide nucléique est effectuée pour chaque compartiment en utilisant des produits d'amplification marqués par fluorescence.

**11.** Procédé selon l'une quelconque des revendications précédentes, dans lequel le procédé de détection à deux composants comprend l'utilisation de multiples composants de liaison spécifiques à l'analyte comprenant de codes-barres d'identification de molécules d'acide nucléique, à identifiant moléculaire unique et utilise un essai compartimenté, dans lequel une amplification d'acide nucléique est effectuée pour chaque compartiment produisant des codes-barres d'acide nucléique identifiant des molécules liées, à identifiant moléculaire unique, les compartiments sont réunis dans un pool commun et une technique de séquençage d'acide nucléique parallèle est utilisée pour produire le signal dépendant de la concentration des complexes bicomposant/analyte.

**12.** Procédé selon l'une quelconque des revendications précédentes 10 ou 11, dans lequel la technique de séquençage parallèle d'acide nucléique est une technique de séquençage de nouvelle génération (NGS).

**13.** Kit pour déterminer les constantes de dissociation dans un procédé de détection bicomposant comprenant :

a. un tampon de réaction pour au moins deux composants de liaison spécifiques à un analyte à des concentrations connues pour mettre en œuvre un procédé de détection à deux composants comprenant la mise en contact desdits deux composants de liaison spécifique avec une solution contenant un analyte pour produire un signal qui dépend de la concentration de complexes bicomposant/analyte formés dans ladite solution,
b. des données de référence sur une relation de constantes de dissociation pour ledit procédé de détection à deux composants qui est une fonction mathématique pour la relation entre kd1 et kd2 en fonction dudit signal reflétant la concentration de complexes bicomposant/analyte et la concentration d'analytes et/ou de composants de liaison spécifiques à l'analyte,
c. éventuellement des instructions pour préparer une ou plusieurs dilutions dudit échantillon et appliquer le procédé de détection à deux composants à l'échantillon et la ou les dilutions en utilisant des facteurs de dilutions définis,
d. un programme informatique qui, lorsqu'il est exécuté sur un ordinateur, est configuré pour exécuter les étapes de calcul consistant à déterminer les constantes de dissociation kd1 et kd2 dudit composant de liaison spécifiques à l'analyte à l'aide dudit signal détecté dans l'échantillon et dans la ou les dilutions en tant qu'entrée contraignante pour un ajustement mathématique pour ladite relation de constantes de dissociation à des concentrations d'analyte et/ou de composants de liaison spécifiques à l'analyte différentes.

**Fig. 1**

**Fig. 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H06109740 A
- EP 0576879 A2
- CN 106226516 B
- EP 2837937 A1
- EP 1460414 A1
- EP 3224360 A
- US 5213961 A
- US 4816567 A
- US 9209965 W
- WO 9413804 A

**Non-patent literature cited in the description**

- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **DIEFFENBACH, C. W. ; G. S. DVEKSLER.** PCR Primer, a Laboratory Manual. Cold Spring Harbor Press, 1995
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **MORRISON et al.** *Proc. NatL. Acad Sci. USA,* 1984, vol. 81, 6851-6855
- **WARD, E.S.** *Nature,* vol. 341, 544-546
- **BIRD.** *Science,* 1988, vol. 242, 423-426
- **HUSTON et al.** *PNAS USA,* 1988, vol. 85, 5879-5883
- **P. HOLLINGER.** *Proc. Natl. Acad. Sci. USA,* 1993, vol. 90, 6444-6448
- **NYGREN, P. A.** *FEBS J,* 2008, vol. 275 (1 1), 2668-76
- **SKERRA, A.** *FEBS J,* 2008, vol. 275 (1 1), 2677-83
- **WATT, P. M.** *Nat Biotechnol,* 2006, vol. 24 (2), 177-83
- **BLANK ; BLIND.** *Current Opin. Chem. Biol.,* 2005, vol. 9, 336-342
- **LIU ; LU.** *J Am Chem Soc,* 2003, vol. 125, 6642-6643
- **MENGER et al.** Handbook of Experimental Pharmacology. 2006, 359-373
- **ELLINGTON ; SZOSTAK.** *Nature,* 1990, vol. 346, 818-822
- **JENISON et al.** *Science,* 1994, vol. 263, 1425-1429
- **GEIGER et al.** *Nucl. Acids Res.,* 1996, vol. 24, 1029-1036
- **SCHUERER et al.** *Bioorg. Med. Chem.,* 2001, vol. 92, 2557-2563
- **KIM et al.** *Biosens. Bioelectron.,* 2007, vol. 22, 2525-2531
- **BALDRICH et al.** *Anal Chem.,* 2004, vol. 76 (23), 7053-63
- **TOMBELLI et al.** *Biomolec Eng.,* 2007, vol. 24, 191-200
- **PINHEIRO et al.** *Analytical Chemistry,* vol. 84 (2), 1003-11
- **SCHUSTER.** Next-generation sequencing transforms today's biology. *Nature Methods,* 2008, vol. 5, 16-18
- **METZKER.** Sequencing technologies the next generation. *Nat Rev Genet.,* January 2010, vol. 11 (1), 31-46
- Hook''-calibration of GeneChip-microarrays: Theory and algorithm. **BINDER H. et al.** Algorithms for molecular biology. Biomed Central LTD, 29 August 2008, vol. 3, 12
- **DOUGLASS E. F. et al.** A Comprehensive Mathematical Model for Three-Body Binding Equilibria. *Journal of the American Chemical Society,* 16 April 2013, vol. 135 (16
- **FRIGUET, B. ; CHAFFOTTE, A. F. ; DJAVADI-OHANIANCE, L. ; GOLDBERG, M. E.** Measurements of the true affinity constant in solution of antigen-antibody complexes by enzyme-linked immunosorbent assay. *Journal of Immunological Methods,* 1985, vol. 77 (2), 305-319, http://www.ncbi.nlm.nih.gov/pubmed/3981007
- **KARAKUS, U. ; THAMAMONGOOD, T. ; CIMINSKI, K. ; RAN, W. ; GÜNTHER, S. C. ; POHL, M. O. ; STERTZ, S.** MHC class II proteins mediate cross-species entry of bat influenza viruses. *Nature,* 2019, vol. 567 (7746, https://doi.org/10.1038/s41586-019-0955-3

- **MORELL, M. ; VENTURA, S. ; AVILÉS, F. X.** Protein complementation assays: Approaches for the in vivo analysis of protein interactions. *FEBS Letters,* 2009, vol. 583 (11), 1684-1691, https://doi.org/10.1016/j.febslet.2009.03.002
- **PAREKH, S. ; ZIEGENHAIN, C. ; VIETH, B. ; ENARD, W. ; HELLMANN, I.** zUMIs: A fast and flexible pipeline to process RNA sequencing data with UMIs. *BioRxiv,* 2017, 2-7, https://doi.org/10.1101/153940
- **PFLEGER, K. D. G. ; SEEBER, R. M. ; EIDNE, K.A.** Bioluminescence resonance energy transfer (BRET) for the real-time detection of protein-protein interactions. *Nature Protocols,* 2006, vol. 1 (1), 337-345, https://doi.org/10.1038/nprot.2006.52
- **QUAN, P. L. ; SAUZADE, M. ; BROUZES, E.** DPCR: A technology review. *Sensors (Switzerland,* 2018, vol. 18 (4, https://doi.org/10.3390/s18041271
- **REY E. G. et al.** Mitigating the Hook Effect in Lateral Flow Sandwich Immunoassays Using Real-Time Reaction Kinetics. *ANALYTICAL CHEMISTRY,* 2017, vol. 89 (9), 5095-5100
- **ROSSANT C. J. et al.** Versatility of homogeneous time-resolved fluorescence resonance energy transfer assays for biologic drug discovery. *Journal of Biomolecular Screening Society for Laboratory Automation and Screening,* 2015
- **XIONG Y. et al.** Development of a novel immunoassay to detect interactions with the transactivation domain of p53: application to screening of new drugs. *SCIENTIFIC REPORTS,* 2017, vol. 7 (1
- **SMITH, A. M. ; HEISLER, L. E. ; MELLOR, J. ; KAPER, F. ; THOMPSON, M. J. ; CHEE, M. ; NISLOW, C.** Quantitative phenotyping via deep barcode sequencing. *Genome Research,* 2009, vol. 19 (10), 1836-1842, https://doi.org/10.1101/gr.093955.109
- **SÖDERBERG, O. ; GULLBERG, M. ; JARVIUS, M. ; RIDDERSTRÅLE, K. ; LEUCHOWIUS, K.-J. ; JARVIUS, J. ; LANDEGREN, U.** Direct observation of individual endogenous protein complexes in situ by proximity ligation. *Nature Methods,* 2006, vol. 3 (12), 995-1000, https://doi.org/10.1038/nmeth947
- **TAOUJI, S. ; DAHAN, S. ; BOSSE, R. ; CHEVET, E.** Current Screens Based on the AlphaScreen™ Technology for Deciphering Cell Signalling Pathways. *Current Genomics,* 2009, vol. 10 (2), 93-101, https://doi.org/10.2174/138920209787847041
- Förster resonance energy transfer immunoassays using engineered proteins for breast cancer biomarker detection. **WU et al.** Ph.D. Thesis. Université Paris-Saclay, 2018
- **ZORBA A. et al.** Delineating the role of cooperativity in the design of potent PROTACs for BTK. *Proceedings of the national academy of sciences,* 16 July 2018, vol. 115 (31